# EUROPÄISCHE PATENTSCHRIFT

(11) **EP 1 847 571 B1**
(45) Veröffentlichungstag und Bekanntmachung des Hinweises auf die Patenterteilung: **25.12.2013**
(21) Anmeldenummer: 07006506.5
(22) Anmeldetag: 29.03.2007
(51) Int. Cl.: C09B 67/20, A23L 1/275, C09C 1/00

(54) **Pigment comprising a plate-shaped substrate**
Pigment umfassend ein plättchenförmiges Substrat
Colorant comportant un substrat en forme de plaque

(30) Priorität: 21.04.2006 EP 06008237
(43) Veröffentlichungstag der Anmeldung: 24.10.2007
(73) Patentinhaber: Merck Patent GmbH, 64293 Darmstadt (DE)
(72) Erfinder: Handrosch, Carsten, Dr., 64367 Mühltal (DE); Lorenz, Carsten, 68649 Gross-Rohrheim (DE)

(56) Entgegenhaltungen:
- EP-A- 1 045 014
- EP-A- 1 481 658
- WO-A-03/063616
- DE-A1- 4 225 357
- US-A- 6 113 683

## Beschreibung

Die vorliegende Erfindung betrifft ein Pigment umfassend ein plättchenförmiges Substrat, dadurch gekennzeichnet, dass das Substrat ausgewählt ist aus Glasplättchen, SiO₂-Plättchen, Al₂O₃-Plättchen, künstlichem oder natürlichem plättchenförmigen Eisenoxid, künstlichem oder natürlichem Graphit und/oder plättchenförmigen Metallen und sich auf dem Substrat eine Anion-bindende Schicht umfassend ein schichtförmiges Doppelhydroxid (LDH) befindet, wobei die Anion-bindende Schicht ein Anion-bildendes organisches, anorganisches und/oder metallorganisches Farbmittel aus der Gruppe C.I. Acid-Farbstoffe, C.I. Reaktiv-Farbstoffe, C.I. Direkt-Farbstoffe, C.I. Mordant-Farbstoffe, C.I. Solubilised Sulphur Red 11, Fluoreszenz-Farbstoffe, FD&C Yellow 5 (Tartrazin), FD&C Yellow 6 (Sunset Yellow FCF), FD&C Yellow 10, FD&C Red 3 (Erythrosin), FD&C Red 6 (Litholrubin B), FD&C Red 7 (Litholrubin BN), FD&C Red 21, FD&C Red 27, FD&C Red 28 (Floxine B), FD&C Red 33, C.I. Natural Red 33, FD&C Red 36, FD&C Red 40, Carmine, FD&C Blue 1 (Brilliant Blue FCF), C.I. Natural Green 3 (E141), FD&C Blue, FD&C Black 1 (Brilliant Black) enthält. Weiterhin betrifft die vorliegende Erfindung ein Verfahren zur Herstellung des erfindungsgemäßen Pigmentes sowie dessen Verwendung in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

Der Einsatz von Glanz- oder Effektpigmenten, die Interferenzerscheinungen aufweisen, ist weit verbreitet. Bei AutomobilLacken, dekorativen Beschichtungen aller Art sowie bei der Einfärbung von Kunststoffen, bei Anstrich- und Druckfarben, insbesondere Farben für den Sicherheitsdruck, sowie bei Anwendungen in der dekorativen Kosmetik sind derartige Pigmente unverzichtbar geworden. In der sie umgebenden Matrix richten sich diese Pigmente idealerweise parallel zur Oberfläche der Beschichtung aus und entfalten durch ein kompliziertes Zusammenspiel von Interferenz, Reflexion und Absorption des auffallenden Lichtes ihre optische Wirkung. Dabei stehen für die verschiedenen Anwendungsfälle eine brillante Farbgebung, Wechsel zwischen verschiedenen Farben in Abhängigkeit vom Betrachtungswinkel, so genannte Farbflops, oder wechselnde Helligkeitseindrücke im Mittelpunkt des Interesses.

Herkömmliche Effektpigmente zeigen aber vielfach nur eine geringe Farbsättigung und ein begrenztes Farbspektrum. Zur Erweiterung des Farbspektrums wurde in EP 1 481 658 vorgeschlagen, natürliche Schichtsilikate, wie z.B. Glimmer, mit einer Hydrotalcit-artigen Schicht eines Doppelhydroxids enthaltend Farbstoffanionen zu beschichten. Die im Stand der Technik genannten Pigmente haben aber den Nachteil, dass die Farben vielfach nicht rein und nicht kräftig genug sind. Dies sind jedoch wesentliche Bedingungen, um in den einzelnen Anwendungen optimale Farbgebungsergebnisse erzielen zu können.

Es ist daher die Aufgabe der vorliegenden Erfindung, ein Pigment bereitzustellen, das sich durch besondere Farbkraft und -stärke sowie durch besonders reine Farben auszeichnet. Darüber hinaus soll ein Pigment bereitgestellt werden, das zusätzlich auch eine hohe Deckkraft aufweisen kann.

Überraschenderweise wird die oben genannte Aufgabe durch ein Pigment der vorliegenden Erfindung gelöst.

Demgemäß ist ein Pigment, wie oben definiert, ein erster Gegenstand der vorliegenden Erfindung.

Ein Pigment auf der Basis der erfindungsgemäß bevorzugt ausgewählten Substrate zeigt, neben der attraktiven Kombination aus Interferenz- und Absorptionsfarbe, eine besonders hohe Farbreinheit bezüglich der Interferenzfarbe des Substrates und der Absorptionsfarbe des organischen, anorganischen und/oder metallorganischen Farbmittels. Weist beispielsweise das Substrat eine Interferenzfarbe auf, so kann sich diese mit der Absorptionsfarbe des organischen, anorganischen und/oder metallorganischen Farbmittels überlagern, so dass ein Hybridpigment erhalten wird, das heißt es wird ein Pigment mit mindestens zwei unterschiedlichen Farbeffekten erhalten, die auf unterschiedlichen physikalischen Gesetzmäßigkeiten beruhen.
Darüber hinaus zeigt das erfindungsgemäße Pigment auf Basis von Glasplättchen zusätzlich einen höheren Glanz als ein Pigment auf Basis von natürlichen Schichtsilikaten. Weiterhin zeigt ein derartiges Pigment einen Glitzereffekt, der mit herkömmlichen Pigmenten in der Art nicht oder nur schwer realisierbar ist. Im Falle von plättchenförmigen Metallen werden farbstarke Pigmente erhalten, die zusätzlich eine hohe Deckkraft aufweisen. Diese spezifische Kombination von hoher Deckkraft und hoher Farbstärke und -reinheit ist mit Metall-Pigmenten aus dem Stand der Technik nicht zu realisieren. Durch die Fixierung des Farbmittel-Anions durch Interkalation in die positiv geladenen Zwischenschichten des bevorzugt eingesetzten Doppelhydroxids wird gegenüber einer einfachen, adsorptiven Bindung eine Erhöhung der Stabilität erreicht. Dies macht sich in einer deutlich höheren Beständigkeit gegenüber Ausbluten, das heißt deutlich geringere Freisetzung des Farbmittels, bemerkbar. Zusätzlich zeigen das erfindungsgemäße Pigment gegenüber dem reinen Farbmittellon bzw. einer einfachen physikalischen Mischung aus Interferenz-Pigment und Farbmittel-Salz ein deutlich besseres Dispergierverhalten.

Aufgrund der vorteilhaften Eigenschaften eignet sich das erfindungsgemäße Pigment universell für eine große Anzahl unterschiedlichster Anwendungen. Gegenstand der vorliegenden Erfindung ist demgemäß auch die Verwendung dieses Pigmentes in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

Das erfindungsgemäße Pigment basiert auf einem plättchenförmigen Substrat ausgewählt aus Glasplättchen, SiO₂-Plättchen, Al₂O₃-Plättchen, künstlichem oder natürlichem plättchenförmigen Eisenoxid, künstlichem oder natürlichem Graphit und/oder plättchenförmigen Metallen. Geeignete Metallplättchen können unter anderem aus Aluminium, Titan, Bronze, Stahl oder Silber bestehen, vorzugsweise aus Aluminium und/oder Titan. Die Metallplättchen können dabei durch entsprechende Behandlung passiviert sein.

Vorzugsweise handelt es sich bei dem plättchenförmigen Substrat um Glasplättchen, SiO₂-Plättchen oder Al₂O₃-Plättchen und, aufgrund ihrer besonders glatten Oberfläche und ihres sehr hohen Reflexionsvermögens, ganz besonders bevorzugt um Glasplättchen.

Bei den bevorzugt eingesetzten SiO₂-Plättchen handelt es sich um synthetische SiO₂-Plättchen, die über eine einheitliche Schichtdicke verfügen und vorzugsweise gemäß der internationalen Anmeldung WO 93/08237 auf einem endlosen Band durch Verfestigung und Hydrolyse einer Wasserglaslösung hergestellt werden. Unter einheitlicher Schichtdicke wird dabei eine Schichtdickentoleranz von 3 bis 10%, bevorzugt von 3 bis 5 % der Gesamttrockenschichtdicke der Partikel verstanden. Die plättchenförmigen Siliziumdioxidpartikel liegen im Allgemeinen in amorpher Form vor. Synthetische Plättchen dieser Art haben gegenüber natürlichen Materialien, wie z.B. Glimmer, den Vorteil, dass die Schichtdicke im Hinblick auf die gewünschten Effekte eingestellt werden kann und die Schichtdickentoleranz begrenzt ist.

Die ganz besonders bevorzugten Glasplättchen können aus allen dem Fachmann bekannten Glastypen bestehen, insbesondere Ca-Al-Borosilikatgläsern, wie z.B. Fensterglas, C-Glas, E-Glas, ECR-Glas, Duran^{®}-Glas, Laborgeräteglas oder optisches Glas. Insbesondere bevorzugt ist E-Glas oder ECR-Glas. Der Brechungsindex der Glasplättchen liegt vorzugsweise bei 1,45-1,80, insbesondere bei 1,50-1,70. Ferner sind als bevorzugte Substrate Glasplättchen aus dotiertem Glas geeignet. Als Dotierstoffe sind z.B. Fe, Bi, La, Nb, Ba, Ti, V, Ce, Au und Cu bzw. Gemische daraus, geeignet. Durch die Dotierung sind Gläser mit speziellen Eigenschaften, wie z.B. einem hohen Brechungsindex bis 2,3 oder starker Eigenfarbe einsetzbar.

Als Substrat kommen demnach plättchenförmige Substrate auf Basis von Ca-Al-Borosilikat (z.B. RONASTAR® von Firma Merck KGaA), SiO₂ (z.B. COLORSTREAM® von Firma Merck KGaA), Al₂O₃ (z.B. XIRALLIC® von Firma Merck KGaA), natürlichem blättchenförmigem Eisenoxid (z.B. MIOX® von Fa. Kärntner Montan Industrie), Graphit, künstlichem plättchenförmigem Eisenoxid (z.B. TAROX® von Firma Titan Kogyo), oder metallischem Aluminium in Frage.

Die Größe des Basissubstrates ist an sich nicht kritisch und kann auf den jeweiligen Anwendungszweck abgestimmt werden. Der Durchmesser des plättchenförmigen Substrates liegt üblicherweise zwischen 1 und 500 µm, vorzugsweise zwischen 5 und 200 µm und insbesondere 10-150 µm. Bevorzugte kleinere Partikelgrößen sind weiterhin solche im Bereich von 1-100 µm, insbesondere 5-60 µm und 1-15 µm. Ihre Dicke beträgt zwischen 0.1 und 5 µm und bevorzugt 0.1 bis 1 µm. Das mittlere Aspektverhältnis des plättchenförmigen Substrates, das heißt das Verhältnis vom mittleren Längenmesswert, der hier dem mittleren Durchmesser entspricht, zum mittleren Dickenmesswert, beträgt üblicherweise 5 bis 200, bevorzugt 20 bis 150 und besonders bevorzugt 30 bis 120.

Das Substrat kann mit Ionen oder Elementen dotiert sein, weiche zu einer Färbung des Substrates führen. Darüber hinaus kann das Substrat auch mit Ionen oder Elementen dotiert sein, welche, außer/neben der Farbe, eine bestimmte physikalische Eigenschaft des Substrates hervorrufen, z.B. ein erhöhter Brechungsindex, Leitfähigkeit, Fluoreszenz, Phosphoreszenz, Magnetismus, NLO-Eigenschaften, IR-Reflexion etc. Dabei sind die aufgeführten, durch lonen- oder Elementdotierung induzierten, physikalischen Eigenschaften nur zur Erläuterung der vorliegenden Erfindung gedacht, ohne sie zu begrenzen.

In einer weiteren Ausführungsform der vorliegenden Erfindung können auf dem plättchenförmigen Substrat und unterhalb der Anion-bindenden Schicht oder auf der Anion-bindenden Schicht eine oder mehrere transparente, semitransparente und/oder opake Schichten aus Metalloxiden, Metalloxidhydraten, Metallsuboxiden, Metallen, Metallfluoriden, Metallnitriden, Metalloxynitriden oder Mischungen dieser Materialien aufgebracht sein. Die Metalloxid-, Metalloxidhydrat-, Metallsuboxid-, Metall-, Metallfluorid-, Metallnitrid-, Metalloxynitridschichten oder die Mischungen hieraus können niedrig- (Brechzahl < 1.8) oder hochbrechend (Brechzahl ≥ 1.8) sein. Als Metalloxide und Metalloxidhydrate eignen sich alle dem Fachmann bekannten Metalloxide oder Metalloxidhydrate, wie z. B. Aluminiumoxid, Aluminiumoxidhydrat, Siliziumoxid, Siliziumoxidhydrat, Eisenoxid, Eisenoxidhydrat, Zinnoxid, Ceroxid, Zinkoxid, Zirkoniumoxid, Chromoxid, Titanoxid, insbesondere Titandioxid, Titanoxidhydrat sowie Mischungen hieraus, wie z.B. Ilmenit oder Pseudobrookit. Als Metallsuboxide können beispielsweise die Titansuboxide eingesetzt werden. Als Metalle eignen sich z.B. Chrom, Aluminium, Nickel, Silber, Gold, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt werden Metalloxid-, Metall-, Metallfluorid und/oder Metalloxidhydratschichten und ganz besonders bevorzugt Metalloxid- und/oder Metalloxidhydratschichten auf den Träger aufgebracht. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln. Insbesondere bevorzugt sind Schichtpakete aus einer hoch- und einer niedrigbrechenden Schicht, wobei auf dem Träger eines oder mehrere dieser Schichtpakete aufgebracht sein können. Die Reihenfolge der hoch- und niedrigbrechenden Schichten kann dabei an das Substrat angepasst werden, um das Substrat in den Mehrschichtaufbau mit einzubeziehen. In einer weiteren Ausführungsform können die Metalloxid-, Metalloxidhydrat-, Metallsuboxid-, Metall-, Metallfluorid-, Metallnitrid-, Metalloxynitridschichten mit Farbmitteln oder anderen Elementen versetzt oder dotiert sein. Als Farbmittel oder andere Elemente eignen sich beispielsweise organische oder anorganische Farbpigmente wie farbige Metalloxide, z.B. Magnetit, Chromoxid oder Farbpigmente wie z.B. Berliner Blau, Ultramarin, Bismutvanadat, Thenards Blau, oder aber organische Farbpigmente wie z.B. Indigo, Azopigmente, Phthalocyanine oder auch Karminrot oder Elemente wie z.B. Yttrium oder Antimon, aber auch farbige Heteropolyanionen. Die äußere Schicht auf dem Substrat ist in einer bevorzugten Ausführungsform ein hochbrechendes Metalloxid. Diese äußere Schicht kann zusätzlich auf den oben genannten Schichtpaketen oder bei hochbrechenden Trägern Teil eines Schichtpaketes sein und z.B. aus TiO₂, Titansuboxiden, Fe₂O₃, SnO₂, ZnO, ZrO₂, Ce₂O₃, CoO, Co₃O₄, V₂O₅, Cr₂O₃ und/oder Mischungen davon, wie zum Beispiel Ilmenit oder Pseudobrookit, bestehen. TiO₂ ist besonders bevorzugt.
Die genannten beschichteten Substrate zeigen eine oder mehrere, winkelabhängige Interferenzfarben. Besonders intensive Farbeindrücke erreicht man, wenn die Interferenzfarbe des Substrates mit der Absorptivfarbe des Farbmittels übereinstimmt. Unterscheidet sich die Interferenzfarbe des Substrates von der Absorptivfarbe des Farbmittels, so erzielt man attraktive Multicolor-Effekte.

Die Dicke der Metalloxid-, Metalloxidhydrat-, Metallsuboxid-, Metall-, Metallfluorid-, Metallnitrid-, Metalloxynitridschichten oder einer Mischung daraus beträgt üblicherweise 3 bis 300 nm und im Falle der Metalloxid-, Metalloxidhydrat-, Metallsuboxid-, Metallfluorid-, Metallnitrid-, Metalloxynitridschichten oder einer Mischung daraus vorzugsweise 20 bis 200 nm. Die Dicke der Metallschichten beträgt vorzugsweise 4 bis 50 nm.

Im Falle von Glasplättchen als plättchenförmiges Substrat sind diese ganz besonders bevorzugt mit einer SiO₂-Schicht beschichtet. Durch die SiO₂-Belegung wird die Glasoberfläche vor chemischer Veränderung wie Quellung, Auslaugen von Glasbestandteilen oder Auflösung in den aggressiven sauren Belegungslösungen geschützt. Während des Glühprozesses bei der Herstellung dieser Substrate kommt es im Fall der Glasplättchen an der Grenzfläche zwischen Glaskörper und aufgefälltem SiO₂ zu einem innigen Verbund der chemisch verwandten Materialien. Aufgrund der hohen Erweichungstemperatur gibt die aufgefällte SiO₂-Hülle den Substraten auch beim Glühen oberhalb von 700°C die erforderliche mechanische Stabilität. Auch die Haftung der auf die SiO₂-Schichten folgenden Beschichtung(en) auf dem Substrat wird verbessert. Die Dicke der SiO₂-Schicht auf den Glasplättchen kann in Abhängigkeit vom gewünschten Effekt in weiten Bereichen variiert werden. Die Schicht weist Dicken von 5-350 nm, vorzugsweise von 5-150 nm, auf. Für die Steuerung von Glanz und Farbstärke sind Schichtdicken von 30-100 nm bevorzugt. Die SiO₂-Schicht kann auch mit Rußpartikeln, farbigen Heteropolyanionen, anorganischen Farbpigmenten, und/oder Metallpartikeln dotiert sein, sofern diese Dotierung an Luft oder unter Inertgas bis zu Glühtemperaturen von 700 - 800°C stabil ist. Der Anteil an Dotiermittel in der SiO₂-Matrix beträgt dann 1-30 Gew.-%, vorzugsweise 2-20 Gew.-%, insbesondere 5-20 Gew.-%. Auf die SiO₂-Schicht ist in einer besonders bevorzugten Ausführungsform eine Schicht eines hochbrechenden Metalloxids aufgebracht, insbesondere eine TiO₂-Schicht.

Das oben genannte Substrat, das beschichtet oder unbeschichtet sein kann, ist gemäß der vorliegenden Erfindung mit einer Anion-bindenden Schicht, insbesondere umfassend ein schichtförmiges Doppelhydroxid, beschichtet. Derartige bevorzugte Schichten werden auch als "layered double hydroxide"-Schichten (LDH-Schichten) bezeichnet.

Vorzugsweise umfasst das schichtförmige Doppelhydroxid (LDH) ein Doppelhydroxid der allgemeinen Formel

M²⁺₁₋ₓM³⁺ₓ(OH)₂(Zⁿ⁻)_{x/n} ˙ mH₂O

mit 0.2 < x < 0.33, wobei
M³⁺ ausgewählt ist aus Al³⁺, Cr³⁺, Fe³⁺, Ga³⁺, In³⁺, Y³⁺, La³⁺ und/oder Ce³⁺ und
M²⁺ ausgewählt ist aus Ba²⁺, Ca²⁺, Cu²⁺, Mg²⁺, Sr²⁺ und/oder Zn²⁺,
Zⁿ⁻ ein Gegenion der Metall-Salze und/oder ein Anion- oder ein Anionengemisch der Anion-bildenden organischen und/oder anorganischen Farbmittel bedeutet, dabei steht n für die Ladungszahl des Anions.
m ist ein stöchiometrischer Faktor und gibt den Gehalt an Kristallwasser im LDH an. Im Rahmen der vorliegenden Erfindung kann m beispielsweise 1-12 betragen, darüber hinaus aber auch andere Werte, die ganzzahlig oder nicht ganzzahlig sein können.

Beispiele der genannten Doppelhydroxide sind Mg_{0.67}Al_{0.33}(OH)₂(Zⁿ⁻)_{0.33/n} ˙ mH₂O und Mg_{0.67}Fe_{0.33}(OH)₂(Zⁿ⁻)_{0.33/n} ˙ mH₂O, Zn_{0.67}Al_{0.33}(OH)₂(Zⁿ⁻)_{0.33/n} ˙ mH₂O und Zn_{0.67}Fe_{0.33}(OH)₂(Zⁿ⁻)_{0.33/n} ˙ mH₂O, Ca_{0.67}Al_{0.33}(OH)₂(Zⁿ⁻)_{0.33/n} ˙ mH₂O und Ca_{0.67}Fe_{0.33}(OH)₂(Zⁿ⁻)_{0.33/n} ˙ mH₂O. Insbesondere haben sich Mg_{0.67}Fe_{0.33}(OH)₂(Zⁿ⁻)_{0.33/n} ˙ mH₂O, Ca_{0.67}Al_{0.33}(OH)₂(Zⁿ⁻)_{0.33/n,} Zn_{0.67}Fe_{0.33}(OH)₂(Zⁿ⁻)_{0.33/n} ˙ mH₂O und Ca_{0.67}Fe_{0.33}(OH)₂(Zⁿ⁻)_{0.33/n} ˙ mH₂O als besonders vorteilhaft erwiesen.

Vorzugsweise ist M³⁺ = Al³⁺ oder Fe³⁺ und M²⁺ = Mg²⁺, Ca²⁺ oder Zn²⁺. Ganz besonders bevorzugt ist M³⁺ = Fe³⁺. Schichtförmige Doppelhydroxide mit letzterem Kation haben den Vorteil, dass sie keine Aluminium-Kationen enthalten. Dies ist insbesondere für kosmetische Anwendungen von Vorteil, da Aluminium unter Verdacht steht, unter anderem Auslöser für die Parkinson-Krankheit zu sein. Die Schichtdicke der Anion-bindenden Schicht beträgt 0.5-500 nm, insbesondere 1-300 nm.

Die genannte Anion-bindende Schicht enthält Anion-bildende organische, anorganische und/oder metallorganische Farbmittel. Solche Farbmittel können sein: von organischen Farbstoffen oder Pigmenten bzw. von Farbstoff- oder Pigmentvorstufen abgeleitete Anionen oder anorganische bzw. organische Anionen oder Radikalanionen bzw. farbige Heteropolyanionen bzw. ein Gemisch aus den zuvor genannten Farbmitteln.

Für Nicht-Kosmetik-Anwendungen können als Farbmittel prinzipiell alle anionischen bzw. anionbildenden Farbstoffe eingesetzt werden. Geeignet sind dabei insbesondere C.I. Acid-Farbstoffe wie z.B.: C.I. Acid Yellow 13, C.I. Acid Yellow 17, C.I. Acid Yellow 23, C.I. Acid Yellow 25, C.I. Acid Yellow 36, C.I. Acid Yellow 38, C.I. Acid Yellow 42, C.I. Acid Yellow 44, C.I. Acid Yellow 56, C.I. Acid Yellow 65, C.I. Acid Yellow 76, C.I. Acid Yellow 127, C.I. Acid Orange 7, C.I. Acid Orange 10, C.I. Acid Orange 19, C.I. Acid Orange 65, C.I. Acid Orange 67, C.I. Acid Red 1, C.I. Acid Red 13, C.I. Acid Red 14, C.I. Acid Red 32, C.I. Acid Red 37, C.I. Acid Red 38, C.I. Acid Red 42, C.I. Acid Red 88, C.I. Acid Red 119, C.I. Acid Red 131, C.I. Acid Red 138, C.I. Acid Red 154, C.I. Acid Red 249, C.I. Acid Red 299, C.I. Acid Violet 14, C.I. Acid Violet 42, C.I. Acid Violet 43, C.I. Acid Blue 25, C.I. Acid Blue 40, C.I. Acid Blue 43, C.I. Acid Blue 62, C.I. Acid Blue 92, C.I. Acid Blue 113, C.I. Acid Blue 117, C.I. Acid Blue 129, C.I. Acid Green 1, C.I. Acid Green 25, C.I. Acid Green 41, C.I. Acid Black 1, C.I. Acid Black 24, C.I. Acid Black 26, C.I. Acid Black 48, C.I. Acid Black 210, C.I. Acid Black 234, C.I. Acid Brown 14, C.I. Acid Brown 20.

Darüber hinaus sind auch C.I. Reaktiv-Farbstoffe geeignet wie z.B.: C.I. Reactive Yellow 4, C.I. Reactive Yellow 17, C.I. Reactive Orange 1, C.I. Reactive Red 8, C.I. Reactive Red 12, C.I. Reactive Red 23, C.I. Reactive Blue 15, C.I. Reactive Blue 19, C.I. Reactive Blue 216, C.I. Reactive Black 5, C.I. Reactive Black 8, C.I. Reactive Black 31.
Ferner sind geeignet C.I. Direkt-Farbstoffe wie z.B.:
C.I. Direct Yellow 12, C.I. Direct Yellow 27, C.I. Direct Yellow 29, C.I. Direct Yellow 50, C.I. Direct Yellow 86, C.I. Direct Orange 26, C.I. Direct Red 23, C.I. Direct Red 75, C.I. Direct Red 76, C.I. Direct Red 79, C.I. Direct Red 80, C.I. Direct Red 81, C.I. Direct Red 250, C.I. Direct Blue 78, C.I. Direct Blue 86, C.I. Direct Blue 93, C.I. Direct Blue 106, C.I. Direct Green 26, C.I. Direct Black 19, C.I. Direct Black 22, C.I. Direct Black 51, C.I. Direct Black 150, C.I. Direct Black 151, C.I. Direct Black 166, C.I. Direct Black 168. Schließlich können auch C.I. Mordant-Farbstoffe, wie z.B.: C.I. Mordant Yellow 1, C.I. Mordant Yellow 5, C.I. Mordant Yellow 30, C.I. Mordant Red 7, C.I. Mordant Red 19, C.I. Mordant Red 30, C.I. Mordant Blue 7, C.I. Mordant Blue 13, C.I. Mordant Black 3, C.I. Mordant Black 9, C.I. Mordant Black 11, C.I. Mordant Brown 33, C.I. Mordant Brown 48, aber auch C.I. Solubilised Sulphur Red 11 und Fluoreszenz-Farbstoffe wie z.B.: C.I. Basic Yellow 40, C.I. Basic Red 12, C.I. Solvent Yellow 94 eingesetzt werden.

Bevorzugt eingesetzt werden die Anionen der organischen Farbstoffe und Pigmente bzw. deren Vorstufen und Gemische, welche in Kosmetik-Anwendungen zugelassen sind. Beispiele sind: FD&C Yellow 5 (Tartrazin), FD&C Yellow 6 (Sunset Yellow FCF), FD&C Yellow 10, FD&C Red 3 (Erythrosin), FD&C Red 6 (Litholrubin B), FD&C Red 7 (Litholrubin BN), FD&C Red 21, FD&C Red 27, FD&C Red 28 (Floxine B), FD&C Red 33, C.I. Natural Red 33, FD&C Red 36, FD&C Red 40, Carmine, FD&C Blue 1 (Brilliant Blue FCF), C.I. Natural Green 3 (E141), FD&C Blue, FD&C Black 1 (Brilliant Black).

Es besteht insbesondere auch die Möglichkeit Gemische von mindestens 2 Farbmittelanionen über die Anion-bindende Schicht auf dem Substrat zu fixieren. Hierbei ergeben sich unzählige Farbvarianten.

Der Anteil der Anion-bildenden organischen und/oder anorganischen Farbmittel beträgt 0.01 bis 30 Gew.-%, bezogen auf das Gesamtpigment, insbesondere 0.5 bis 10 Gew.-%.

In einer weiteren Ausführungsform der vorliegenden Erfindung ist auf dem Pigment zusätzlich eine stabilisierende anorganische und/oder organische Beschichtung aufgebracht. Diese Nachbeschichtung erhöht, in Abhängigkeit vom Einsatzgebiet, die Licht-, Wasser- und Wetterstabilität. Dabei wird auch die Ausblutbeständigkeit des Produktes weiter erhöht. Beispiele für derartige Beschichtungen finden sich z.B. in DE 22 15 191, DE 31 51 354, DE 33 34 598, EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 EP 0 090 259, EP 0 634 459, WO 99/57204, WO 96/32446, WO 99/57204, US 5,571,851, WO 01/92425. oder EP 0 465 805, deren Offenbarung hiermit unter Bezugnahme mit eingeschlossen ist. Als anorganische Materialien für die Nachbeschichtung eignen sich die Oxide und/oder Oxidhydrate von Al, Si, Zr, Ce, Zn, Fe und/oder Mischungen hieraus, vorzugsweise werden die Oxide und/oder Oxidhydrate von Al, Ce, Zn, Zr und/oder Si eingesetzt. Die genannten Schichten können als einzelne Schichten der jeweiligen Oxide und/oder Oxidhydrate aber auch als Mischschichten vorliegen. Darüber hinaus können neben den alleinig abgeschiedenen Oxiden auch Mischungen von Oxiden mit Sulfaten, Phosphaten und/oder Boraten eingesetzt werden. Beispiele für Sulfate sind ZnSO₄, CaSO₄, für Phosphate AIPO₄, CePO₄ und für Borate AIBO₄.

Schichten dieser Materialien zeichnen sich durch hohe Transparenz, fehlende oder geringe Eigenfarbe und hohen Glanz aus, so dass die coloristischen Eigenschaften des Pigmentes nicht verändert werden. Insgesamt sind die jeweiligen Anteile für die zusätzliche stabilisierende Beschichtung so auszuwählen, dass die optischen Eigenschaften des erfindungsgemäßen Pigmentes nicht wesentlich beeinflusst werden.

Die gegebenenfalls aufgebrachte organische Beschichtung agiert als Kupplungsreagenz und kann aus Organosilanen, -aluminaten, -titanaten und/oder -zirkonaten der allgemeinen Formel

X₄₋ₙ₋ₘZ-Rₙ(-B-Y)ₘ

mit X = OH, Halogen, Alkoxy, Aryloxy
Z = Si, Al, Ti, Zr
R = Alkyl, Phenyl oder Wasserstoff
B = organische, zumindest bifunktionelle Gruppe (Alkylen, Alkylenoxyalkylen)
Y = Amino-, substituierte Amino-, Hydroxy-, Hydroxyalkyl-, Siloxan-, Acetoxy, Isocyanat-, Vinyl-, Acryloyl-, Epoxy-, Epoxypropyloxy-, Imidazol- oder Ureidogruppe
n, m = 0,1,2,3 mit n+m ≤ 3 bestehen.

Die Kupplungsreagenzien bestehen aus einer Ankergruppe (X₄₋ₙ₋ₘZ), die an die Oberfläche bindet, wenigstens einer hydrophoben Gruppe (R,B) sowie einer oder mehrerer funktioneller Gruppe (Y). Vorzugsweise handelt es sich bei den Kupplungsreagenzien um Verbindungen mit Z = Si. Bevorzugt besteht die Ankergruppe aus Alkoxysilanen, die durch hydrolytische Reaktionsbedingungen in entsprechende Hydroxygruppen überführt werden können. Letztere können an die Oberfläche des Pigmentes binden und die Verankerung über Sauerstoffbrücken bewirken. Darüber hinaus können auch Mischungen verschiedener Kupplungsreagenzien eingesetzt werden, die als Mischung oder einzeln aufgebracht werden können.

Durch die Wahl geeigneter funktioneller Gruppen kann die organische Beschichtung dem Einsatzmedium angepasst werden. Darüber hinaus können durch Reaktion der funktionellen Gruppen mit entsprechenden Funktionalitäten in den Applikationsmedien zusätzliche Bindungen zwischen Pigment und Medium über das Kupplungsreagenz erzeugt werden. In einer besonderen Ausführungsform wird die Oberfläche des erfindungsgemäßen Pigmentes mit einer dem Einsatzmedium angepassten Kombination von organischen Funktionalitäten modifiziert. Hierzu eignet sich auch der Einsatz von Mischungen verschiedener Kupplungsreagenzien innerhalb der organischen Beschichtung. Die Hydrophobie der Pigmentoberfläche kann durch Integration von alkylhaltigen Kupplungsreagenzien wie z. B. Alkylsilanen angepasst werden. Neben den Organosilanen ist auch der Einsatz ihrer Hydrolysate sowie homogener und heterogener Oligomere und/oder Polymere bevorzugt, die ebenfalls alleinig oder in Kombination mit Silanen, Zirkonaten, Aluminaten, Zirkonaluminaten und/oder Carboxyzirkonaluminaten als organische Beschichtung eingesetzt werden können. Im Besonderen bevorzugt ist eine organische Beschichtung mit Mischungen verschiedener Kupplungsreagenzien, insbesondere mit voneinander unterschiedlichen funktionellen Gruppen Y, die eine besondere Anwendungsbreite gewährleistet.

Beispiele für Organosilane sind Propyltrimethoxysilan, Propyltriethoxysilan, Isobutyltrimethoxysilan, n-Octyltrimethoxysilan, i-Octyltrimethoxysilan, n-Octyltriethoxysilan, n-Decyltrimethoxysilan, Dodecyltrimethoxysilan, Hexadecyltrimethoxysilan, Vinyltrimethoxysilan, vorzugsweise n-Octyltrimethoxysilan und n-Octyltriethoxysilan. Als oligomere, alkoholfreie Organosilanhydrolysate eignen sich unter anderem die unter dem Handelsnamen "Dynasylan^{®}" von der Fa. Sivento vertriebenen Produkte, wie z. B. Dynasylan HS 2926, Dynasylan HS 2909, Dynasylan HS2907, Dynasylan HS 2781, Dynasylan HS 2776, Dynasylan HS 2627. Darüber hinaus eignet sich oligomeres Vinylsilan als auch Aminosilanhydrolysat als organische Beschichtung. Funktionalisierte Organosilane sind beispielsweise 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan, 1,3-bis(3-glycidoxypropyl)-1,1,3,3,-tetramethyldisiloxan, Ureidopropyltriethoxysilan, bevorzugt sind 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan. Beispiele für polymere Silansysteme sind in WO 98/13426 beschrieben und werden z. B. von der Fa. Sivento unter dem Warenzeichen Hydrosil^{®} vertrieben.

Die Menge der organischen Beschichtung beträgt zwischen 0.2 und 5 Gew.-%, bezogen auf das Pigment, vorzugsweise 0.5 bis 2 Gew.-%.

Ein weiterer Gegenstand der vorliegenden Erfindung ist ein Verfahren zur Herstellung des erfindungsgemäßen Pigmentes wobei eine Suspension von plättchenförmigem Substrat, Metallkation-Salzen, Farbmittelsalzen sowie Laugen und/oder Laugenvorstufen (z.B. Harnstoff) in einem Lösungsmittel oder Lösungsmittelgemisch bei einer Temperatur von 10-120°C gerührt wird, so dass sich auf dem Substrat die Anion-bindende Schicht bildet, bevorzugt umfassend ein schichtförmiges Doppelhydroxid, wobei die Anion-bindende Schicht Anion-bildende organische, anorganische und/oder metallorganische Farbmittel enthält, anschließend das Produkt abgetrennt, gewaschen, getrocknet und gegebenenfalls gesiebt wird.

Im einfachsten Falle werden das Substrat, die Metallkation-Salze und Farbmittel-Salz(e) zusammen mit Lauge und/oder Laugenvorstufen in einer Suspension vorgelegt und abhängig von der Art des LDH und des eingesetzten Farbmittel-Salzes bei einer Temperatur von 10-120°C für 2 bis 48 Stunden gerührt. Nach Abkühlen der Reaktionssuspension wird das Produkt abgesaugt und mit einem geeigneten Lösungsmittel gewaschen, bis das Filtrat nahezu farblos abläuft. Der Filterkuchen wird anschließend bei 50-180°C getrocknet und gegebenenfalls auf die gewünschte Feinheit gesiebt.

In einer weiteren Ausführung kann eine Lösung der Metallkation-Salze zu einer Suspension aus plättchenförmigem Substrat, Farbmittelsalzen sowie Laugen und/oder Laugenvorstufen zugegeben werden. Dies bedeutet im einfachsten Falle, dass das Substrat, Lauge und/oder Laugenvorstufen und Farbmittel-Salz(e) in einer Suspension vorgelegt werden. Bei Raumtemperatur wird anschließend eine Lösung der Metallkation-Salze zugetropft. Nach Beendigung der Zugabe wird das Reaktionsgemisch auf 30-100°C geheizt und abhängig von der Art des LDH und des Farbmittels bei dieser Temperatur für 2 bis 48 Stunden gerührt. Nach Abkühlen der Reaktionssuspension wird das Produkt abgesaugt und mit einem geeigneten Lösungsmittel gewaschen, bis das Filtrat nahezu farblos abläuft. Der Filterkuchen wird anschließend bei 50-180°C getrocknet und gegebenenfalls auf die gewünschte Feinheit gesiebt.

Als Laugen eignen sich wässrige Lösungen von NaOH, KOH oder NH₃, aber auch Laugenvorstufen, wie z.B. Harnstoff, welche im Reaktionsmedium, z.B. durch Hydrolyse, erst die eigentliche Lauge freisetzen. Der pH-Wert bei der Umsetzung (gemeint ist hier der pH-Werts-Verlauf während der gesamten Reaktion) liegt üblicherweise im Bereich von 2 bis 13, insbesondere von 3 bis 11.

Als Metallkation-Salze eignen sich grundsätzlich alle Salze mit Al³⁺, Cr³⁺, Fe³⁺, Ga³⁺, In³⁺, Y³⁺, La³⁺, Ce³⁺, Ba²⁺, Ca²⁺, Cu²⁺, Mg²⁺, Sr²⁺ und/oder Zn²⁺ Kationen, aus denen die Anion-bindende Schicht, insbesondere umfassend ein schichtförmiges Doppelhydroxid, hergestellt werden kann. Insbesondere handelt es sich bei den genannten Metallkation-Salzen um die entsprechenden Halogenide, insbesondere Chloride, Bromide, lodide. Es sind aber auch Metallkation-Salze mit Sulfat- oder Nitrat-Anionen geeignet. Ganz besonders bevorzugt werden die entsprechenden Chloride eingesetzt.

In einer alternativen, 2-stufigen Ausführungsform zur Herstellung des erfindungsgemäßen Pigmentes wird eine Suspension von plättchenförmigem Substrat, Metallkation-Salzen sowie Laugen und/oder Harnstoff in einem Lösungsmittel oder Lösungsmittelgemisch bei einer Temperatur von 10-120°C gerührt, so dass sich auf dem Substrat die Anion-bindende Schicht bildet, insbesondere umfassend ein schichtförmiges Doppelhydroxid, danach wird das Zwischenprodukt abgetrennt, gewaschen, getrocknet bei 50-300°C und gegebenenfalls gesiebt. Dieses mit LDH belegte Substrat wird anschließend zu einer Lösung der Anion-bildenden organischen, anorganischen und/oder metallorganischen Farbmittel unter Rühren hinzu gegeben. Bei dieser Verfahrensvariante kommt es zu einem lonenaustausch, wobei das Anion Zⁿ⁻ des Anion-bindenden Doppelhydroxids gegen das Anion-bildende Farbmittel ausgetauscht wird. Nach Abkühlen der Reaktionssuspension wird das Produkt abgesaugt und gewaschen. Der Filterkuchen wird bei 40-100°C getrocknet. Das trockene Pigment kann anschließend eventuell vermahlen und/oder gesiebt werden.

Bei der letztgenannten Methode sollten als Metallsalze vorzugsweise entsprechende Chloride oder Nitrate eingesetzt werden, da diese beiden Anionen im anschließenden Schritt am Schnellsten gegen das Farbmittelanion ausgetauscht werden. Diese 2-stufige Methode eignet sich insbesondere auch gut für temperaturempfindliche Farbmittel, weil im Vergleich zu den anderen Verfahren das farbmittelhaltige Endprodukt bei deutlich niedrigerer Temperatur getrocknet werden kann.

Bei dieser 2-stufigen Synthese-Variante hat es sich darüber hinaus auch als vorteilhaft erwiesen, das Zwischenprodukt mit der auf dem Substrat gebildeten Anion-bindenden Schicht, bevorzugt umfassend ein schichtförmiges Doppelhydroxid, vor Zugabe zu der Lösung der Anion-bildenden organischen und/oder anorganischen Farbmittel bei Temperaturen von 300-600°C zu kalcinieren. Dabei wird das vorzugsweise mit LDH belegte Substrat durch Kalcinieren bei 300-600°C zunächst in ein LDO-belegtes Substrat umgewandelt. Das LDO ("layered-double-oxide") bildet anschließend im wässrigen bzw. wasserhaltigen Medium wieder die LDH-Struktur zurück und ermöglicht dabei eine leichtere und vollständigere Interkalation.
In einer alternativen Ausführungsform des zuvor beschriebenen 2-stufigen Verfahrens kann auch zunächst eine Suspension des mit LDH bzw. LDO belegten Substrates hergestellt werden, zu der dann bei 20-70°C unter Rühren eine Lösung des Farbmittel-Anions gegeben wird. Das Gemisch lässt man üblicherweise 2 bis 48 h bei dieser Temperatur rühren. Nach beendeter Fixierung des Farbmittel-Anions wird das Produkt abgesaugt und mit einem geeigneten Lösungsmittel gewaschen, bis das Filtrat nahezu farblos abläuft. Der Filterkuchen wird anschließend bei 40-70°C getrocknet und gegebenenfalls auf die gewünschte Feinheit gesiebt.

Darüber hinaus kann in einem ebenfalls erfindungsgemäßen Verfahren zusätzlich als äußere Schicht eine anorganische und/oder organische Beschichtung aufgebracht werden. Beispiele für derartige Beschichtungsverfahren finden sich unter anderem in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805. Beispiele für anorganische und/oder organische Beschichtungen sowie die damit verbundenen Vorteile sind bereits vorab beim Aufbau des erfindungsgemäßen Pigmentes beschrieben worden. Der Verfahrensschritt der Aufbringung der organischen Beschichtung kann direkt an die anderen Schritte des erfindungsgemäßen Verfahrens angeschlossen werden. Die Aufbringung der Kupplungsreagenzien erfolgt in Lösung bei Temperaturen oberhalb von 60°C, vorzugsweise oberhalb von 70°C. Als Lösungsmittel eignen sich organische Lösungsmittel, Wasser oder Mischungen hieraus, bevorzugt wird Wasser verwendet. Die für die Aufbringung der organischen Beschichtung nötige Reaktionszeit liegt bei mindestens 5 Minuten, vorzugsweise erfolgt sie über einen Zeitraum von 10 bis 90 Minuten, kann aber auch beliebig verlängert werden. Das erhaltene Pigment wird nach für den Fachmann gebräuchlichen Methoden aufgearbeitet und isoliert, z. B. durch Filtration, Trocknung und Siebung.

Das erfindungsgemäße Pigment ist vielfältig einsetzbar. Demgemäß ist die Verwendung des erfindungsgemäßen Pigmentes in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten ebenfalls Gegenstand der vorliegenden Erfindung.

Im Falle von Kosmetika eignet sich das erfindungsgemäße Pigment besonders für Produkte und Formulierungen der dekorativen und pflegenden Kosmetik, wie z.B. Salben, Cremes, Pasten, Nagellacke, farbgebende Puder, Lippenstifte oder Lidschatten, Seifen, Zahnpasten etc. Selbstverständlich kann das erfindungsgemäße Pigment in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliziumdioxid, Ca-Silikate, Gelatine, hochmolekulare Kohlenhydrate und/oder oberflächenaktive Hilfsmittel, etc. Das erfindungsgemäße Pigment enthaltende Formulierungen können dem lipophilen, hydrophilen oder hydrophoben Typ angehören. Bei heterogenen Formulierungen mit diskreten wässrigen und nicht-wässrigen Phasen kann das erfindungsgemäße Pigment in jeweils nur einer der beiden Phasen enthalten oder auch über beide Phasen verteilt sein.

Die pH-Werte der wässrigen Formulierungen können zwischen 5 und 14, bevorzugt zwischen 5 und 11 und besonders bevorzugt zwischen 6 und 9 liegen. Der Konzentration des erfindungsgemäßen Pigmentes in der Formulierung ist keine Grenze gesetzt. Sie kann - je nach Anwendungsfall - zwischen 0.001 (rinse-off-Produkte, z.B. Duschgele) - 99% (z.B. Glanzeffekt-Artikel für besondere Anwendungen) liegen. Das erfindungsgemäße Pigment kann weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erythrolose u.a.) sowie weitere kosmetische Wirkstoffe wie z.B. Bisabolol, LPO, Ectoin, Emblica, Allantoin, Bioflavanoide und deren Derivate.

Organische UV-Filter werden in der Regel in einer Menge von 0.5 bis 10 Gewichtsprozent, vorzugsweise 1-8 %, anorganische Filter von 0.1 bis 30% in kosmetische Formulierungen eingearbeitet.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Als Anwendungsform der kosmetischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, lsothionate, lmidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-ÖI (W/O) oder vom Typ ÖI-in-Wasser (O/W), eine multiple Emulsion, beispielsweise vom Typ Waser-in-ÖI-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Bei Einsatz des Pigmentes in Lacken und Farben sind alle dem Fachmann bekannten Anwendungsbereiche möglich, wie z.B. Pulverlacke, Automobillacke, Druckfarben für den Tief-, Offset-, Sieb- oder Flexodruck sowie für Lacke in Außenanwendungen. Die Lacke und Farben können hierbei beispielsweise strahlungshärtend, physikalisch trocknend oder chemisch härtend sein. Für die Herstellung der Druckfarben oder Flüssiglacke ist eine Vielzahl von Bindern, z.B. auf der Basis von Acrylaten, Methacrylaten, Polyestern, Polyurethanen, Nitrocellulose, Ethylcellulose, Polyamid, Polyvinylbutyrat, Phenolharzen, Maleinharzen, Stärke oder Polyvinylalkohol, Aminharzen, Alkydharzen, Epoxidharzen, Polytetrafluorethylen, Polyvinylidenfluoriden, Polyvinylchlorid oder Mischungen hieraus geeignet, insbesondere wasserlösliche Typen. Bei den Lacken kann es sich um Pulverlacke oder wasser- oder lösemittelbasierte Lacke handeln, wobei die Auswahl der Lackbestandteile dem Allgemeinwissen des Fachmanns unterliegt. Gängige polymere Bindemittel für Pulverlacke sind beispielsweise Polyester, Epoxide, Polyurethane, Acrylate oder Mischungen hieraus.

Darüber hinaus kann das erfindungsgemäße Pigment in Folien und Kunststoffen verwendet werden, so z.B. in Agrarfolien, infrarotreflektierenden Folien und Scheiben, Geschenkfolien, Kunststoffbehältnissen und Formkörpern für alle dem Fachmann bekannten Anwendungen. Als Kunststoffe eignen sich alle gängigen Kunststoffe für die Einarbeitung des erfindungsgemäßen Pigmentes, z.B. Duromere oder thermoplastische Kunststoffe. Die Beschreibung der Anwendungsmöglichkeiten und der einsetzbaren Kunststoffe, Verarbeitungsverfahren und Additive finden sich z.B. in der RD 472005 oder in R. Glausch, M. Kieser, R. Maisch, G. Pfaff, J. Weitzel, Perlglanzpigmente, Curt R. Vincentz Verlag, 1996, 83 ff., deren Offenbarungsgehalt hier mit umfasst ist.

Darüber hinaus eignet sich das erfindungsgemäße Pigment auch für den Einsatz im Sicherheitsdruck und in sicherheitsrelevanten Merkmalen für z.B. fälschungssichere Karten und Ausweise, wie z.B. Eintrittskarten, Personalausweise, Geldscheine, Schecks und Scheckkarten sowie für andere fälschungssichere Dokumente. Im Bereich der Landwirtschaft kann das Pigment zur Einfärbung von Saatgut und anderen Ausgangsgütern verwendet werden, darüber hinaus im Lebensmittelbereich zur Pigmentierung von Lebensmitteln. Zur Pigmentierung von Überzügen in Arzneimitteln wie z.B. Tabletten oder Dragees ist das erfindungsgemäße Pigment ebenfalls einsetzbar.

Das erfindungsgemäße Pigment eignet sich in den oben genannten Anwendungsgebieten ebenso zur Verwendung in Abmischungen mit organischen und/oder anorganischen Farbstoffen und/oder Pigmenten, wie z.B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Plättchen auf Basis von Glimmer, Glas, Al₂O₃, Fe₂O₃, SiO₂, etc. Beispiele und Ausführungsformen der oben genannten Materialien und Pigmentaufbauten finden sich z.B. auch in den Research Disclosures RD 471001 und RD 472005, deren Offenbarungen hiermit unter Bezugnahme mit eingeschlossen sind. Das erfindungsgemäße Pigment kann in jedem Verhältnis mit handelsüblichen Pigmenten und Füllern gemischt werden.

Als Füllstoffe sind z.B. natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen. Sie kann den Anforderungen gemäß z.B. plättchenförmig, sphärisch oder nadelförmig sein.

Das erfindungsgemäße Pigment ist weiterhin geeignet zur Herstellung von fließfähigen Pigmentpräparationen und Trockenpräparaten enthaltend ein oder mehrere erfindungsgemäße Partikel, Bindemittel und optional ein oder mehrere Additive. Unter Trockenpräparate sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0.2-80 mm auf. Die Trockenpräparate finden insbesondere Anwendung bei der Herstellung von Druckfarben und in kosmetischen Formulierungen.

Kosmetika, Lacke, Farben, Kunststoffe, Folien, Dokumente und Ausweise, Saatgut, Lebensmittel oder Arzneimittelüberzüge sowie Pigmentpräparationen und Trockenpräparate enthaltend ein Pigment gemäß der vorliegenden Erfindung sind ein weiterer Gegenstand der vorliegenden Erfindung.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung näher erläutern, ohne sie jedoch zu begrenzen.

### Beispiele

### Beispiel 1:

50 g Glasplättchen (ECR-Glas; Fraktion: 10-100 µm, Substrat-Dicke: 850 nm) werden zu 300 ml Wasser und 150 ml NaOH (0.5 M) zugegeben. Nach 30 min Rühren werden 5.8 g C.I. FD&C Red 6 "Unipure Red LC303" als Farbmittel-Vorstufe zugegeben. 10.15 MgCl₂ x 6H₂O, 6.05 g AlCl₃ x 6 H₂O und 20.95 g Harnstoff werden in je ca. 100 ml Wasser gelöst und ebenfalls hinzugegeben. Es wird für 24 h unter Rückfluss gerührt. Man lässt die Suspension abkühlen, saugt den Rückstand ab und wäscht mit Wasser bis das Filtrat nahezu farblos abläuft. Der Rückstand wird bei 50°C getrocknet.

Man erhält ein Pigment mit brillant roter Pulverfarbe, welches einen deutlichen Glitzer-Effekt bei sehr gutem Deckvermögen zeigt.

### Beispiel 2:

50 g eines roten Interferenzpigmentes (ECR-Glas beschichtet mit ca. 4 Gew.-% SiO₂, ca. 1 Gew.-% SnO₂ und ca. 25 Gew.-% TiO₂; Fraktion: 10-100 µm, Substrat-Dicke: 850 nm) werden zu 190 ml Wasser und 310 ml NaOH (0.5 M) zugegeben. Nach 30 min Rühren werden 5.8 g C.I. FD&C Red 6 "Unipure Red LC303" als Farbmittel-Vorstufe zugegeben. 10.15 MgCl₂ x 6H₂O und 6.05 g AICl₃ x 6 H₂O werden zusammen in ca. 150 ml Wasser gelöst und innerhalb einer Stunde unter Rühren zugegeben. Anschließend wird mit Wasser auf ein Gesamtvolumen von 750 ml aufgefüllt. Es wird für 24 h unter Rückfluss gerührt. Man lässt die Suspension abkühlen, saugt den Rückstand ab und wäscht mit Wasser bis das Filtrat nahezu farblos abläuft. Der Rückstand wird bei 110°C getrocknet.

Man erhält ein Pigment mit brillant roter Pulverfarbe, welche überlagert ist von einer einen roten Interferenzfarbe und dabei einen deutlichen Glitzer-Effekt bei sehr gutem Deckvermögen aufweist.

### Beispiel 3:

a) 50 g eines violetten Interferenzpigmentes (ECR-Glas beschichtet mit ca. 4 Gew.-% SiO₂, ca. 1 Gew.-% SnO₂ und ca. 27 Gew.-% TiO₂; Fraktion: 10-100 µm, Substrat-Dicke: 850 nm) werden zu 190 ml Wasser und 310 ml NaOH (0.5 M) zugegeben. Nach 30 min Rühren wird eine Lösung von 10.15 MgCl₂ x 6H₂O und 6.05 g AlCl₃ x 6 H₂O in 150 ml Wasser innerhalb einer Stunde unter Rühren zugegeben. Anschließend wird mit Wasser auf ein Gesamtvolumen von 750 ml aufgefüllt. Es wird für 24 h unter Rückfluss gerührt. Man lässt die Suspension abkühlen, saugt den Rückstand ab und wäscht mit ca. zwei Liter Wasser. Der Rückstand wird bei 50°C getrocknet.
b) 50 g eines violetten Interferenzpigmentes (ECR-Glas beschichtet mit ca. 4 Gew.-% SiO₂, ca. 1 Gew.-% SnO₂ und ca. 27 Gew.-% TiO₂; Fraktion: 10-100 µm, Substrat-Dicke: 850 nm) werden zu 190 ml Wasser und 310 ml NaOH (0.5 M) zugegeben. Nach 30 min Rühren wird eine Lösung von 10.15 MgCl₂ x 6H₂O und 6.05 g AICl₃ x 6 H₂O in 150 ml Wasser innerhalb einer Stunde unter Rühren zugegeben. Anschließend wird mit Wasser auf ein Gesamtvolumen von 750 ml aufgefüllt. Es wird für 24 h unter Rückfluss gerührt. Man lässt die Suspension abkühlen, saugt den Rückstand ab und wäscht mit ca. zwei Liter Wasser. Der Rückstand wird zunächst bei 110°C getrocknet und anschließend bei 300-600°C kalciniert. Die LDH-Schicht auf dem Substrat wandelt sich dabei in eine "layered double oxide"-Schicht (LDO-Schicht) um.
c) 25 g Zwischenprodukt aus Schritt a) oder 20 g Zwischenprodukt aus Schritt b) werden unter Rühren in 135 ml Wasser suspendiert. Es werden 2.9 g C.I. FD&C Red 6 "Unipure Red LC303" als Farbstoff zugegeben. Der pH-Wert der Mischung wird auf 12 eingestellt. Die Suspension wird 12 - 24 Stunden gerührt, abgesaugt, mit Wasser gewaschen bis das Filtrat nahezu farblos abläuft und das Produkt anschließend bei 50°C getrocknet. Besonders bevorzugt geht man so vor, dass eine möglichst gesättigte, wässrige Lösung von 2.9 g C.I. FD&C Red 6 "Unipure Red LC303" bei pH = 12 vorgelegt wird. In diese werden dann 25 g Zwischenprodukt aus Schritt a) oder 20 g Zwischenprodukt aus Schritt b) unter Rühren suspendiert. Auch bei dieser Vorgehensweise wird die Suspension 12 - 24 Stunden gerührt, abgesaugt, mit Wasser gewaschen bis das Filtrat nahezu farblos abläuft, und das Produkt anschließend bei 50°C getrocknet.

Bei beiden Vorgehensweisen erhält man jeweils ein Pigment mit brillant roter Pulverfarbe, welche überlagert ist von einer einen blaustich-roten bis violetten Interferenzfarbe und dabei einen deutlichen Glitzer-Effekt bei sehr gutem Deckvermögen aufweist.

### Anwendungsbeispiele:

### Eye Shadow Gel

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, Cl 77891 (Titanium Dioxide), Magnesium Hydroxide, Cl 15850, Cl 77492 (Iron Oxides), Silica, Tin Oxide | 15,00 |
| Micronasphere M | Merck KGaA/Rona^{®} | Mica, Silica | 8,00 |
| Carbopol Ultrez 21 | Noveon | ACRYLATES/C10-30 ALKYL ACRYLATE CROSSPOLYMER | 0,40 |
| Citronensäure Monohydrat | Merck KGaA/Rona^{®} | CITRIC ACID | 0,00 |
| Wasser | | AQUA (WATER) | ad 100 |

### Phase B

| **Rohstoff** | **Bezugsquelle** | **INCI** | **[%]** |
|---|---|---|---|
| Glycerin | Merck KGaA/Rona^{®} | GLYCERIN | 3,00 |
| Konservierungsmittel | | | q.s. |
| Triethanolamin | | TRIETHANOLAMINE | 0,70 |
| Wasser | | AQUA (WATER) | 13,00 |

### Phase C

| **Rohstoff** | **Bezugsquelle** | **INCI** | **[%]** |
|---|---|---|---|
| Lubrajel DV | | PROPYLENE GLYCOL, POLYGLYCERYL-METHACRYLATE | 5,00 |

### Herstellung:

Rotes Pigment und Micronasphere^{®} M im Wasser der Phase A dispergieren. Mit einigen Tropfen Citronensäure ansäuern um die Viskosität zu vermindern, Carbopol unter Rühren einstreuen. Nach vollständiger Lösung die vorgelöste Phase B langsam einrühren und anschließend Phase C. Zum Schluss den pH-Wert zwischen 7,0 - 7, 5 einstellen.

### Puder Rouge

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, CI 77891 (Titanium Dioxide), Magnesium Hydroxide, Cl 15850, Cl 77492 (Iron Oxides), Silica, Tin Oxide | 30,00 |
| Talkum | Merck KGaA/Rona^{®} | TALC | 49,50 |
| Magnesiumstearat | Merck KGaA/Rona^{®} | MAGNESIUM STEARATE | 2,50 |
| Kartoffelstärke | Suedstaerke GmbH | SOLANUM TUBEROSUM (POTATO STARCH) | 7,50 |

### Phase B

| **Rohstoff** | **Bezugsquelle** | **INCI** | **[%]** |
|---|---|---|---|
| Isopropylstearat | Cognis GmbH | ISOPROPYL STEARATE | 9,34 |
| Cetylpalmitat | Merck KGaA/Rona^{®} | CETYL PALMITATE | 0,53 |
| Ewalin 1751 | H. Erhard Wagner GmbH | PETROLATUM | 0,53 |
| Propyl-4-hydroxybenzoat | Merck KGaA/Rona^{®} | PROPYLPARABEN | 0,10 |

### Herstellung:

Bestandteile der Phase A zusammen geben und vormischen. Anschließend die Pudermischung unter Rühren tropfenweise mit der geschmolzenen Phase B versetzen. Die Puder werden bei 40-50 bar gepreßt.

### Lippenstift

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, Cl 77891 (Titanium Dioxide), Magnesium Hydroxide, Cl 15850, Cl 77492 (Iron Oxides), Silica, Tin Oxide | 12,00 |
| Ronastar^{®} Purple | Merck | Calcium Aluminum | 3,00 |
| Sparks | KGaA/Rona^{®} | Borosilicate, Cl 77891 (Titanium Dioxide), Silica, Tin Oxide | |

### Phase B

| **Rohstoff** | **Bezugsquelle** | **INCI** | **%]** |
|---|---|---|---|
| Bienenwachs | Merck KGaA/Rona^{®} | Cera Alba (Beeswax) | 8,75 |
| Paracera C44 | Paramelt | COPERNICIA CERIFERA (CARNAUBA WAX), CERESIN | 5,25 |
| Adeps Lanae | Henry Lamotte GmbH | LANOLIN | 3,50 |
| Isopropylmyristat | Cognis GmbH | Isopropyl Myristate | 5,60 |
| Paraffin dickflüssig | Merck KGaA/Rona^{®} | PARAFFINUM LIQUIDUM (MINERAL OIL) | 2,10 |
| Rizinusöl | Henry Lamotte GmbH | RICINUS COMMUNIS (CASTOR OIL) | 59,65 |
| Oxynex K flüssig | Merck KGaA/Rona^{®} | PEG-8, TOCOPHEROL, ASCORBYL PALMITATE ASCORBIC ACID, CITRIC ACID | 0,05 |
| Propyl-4-hydroxybenzoat | Merck KGaA/Rona^{®} | PROPYLPARABEN | 0,10 |

### Herstellung:

Die Bestandteile der Phase B werden auf 75 °C erhitzt und aufgeschmolzen. Die Pigmente der Phase A werden zugegeben und alles gut durchrührt. Die Lippenstiftmasse wird dann in der auf 65 °C temperierten Gießapparatur 15 Minuten gerührt. Die homogene Schmelze wird in die auf 55 °C vorgewärmten Gießform gegossen. Anschließend kühlt man die Formen ab und entfernt die Gießlinge kalt. Nach Erwärmen der Lippenstifte auf Raumtemperatur werden die Lippenstifte kurz abgeflammt.

### Nagellack

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, CI 77891 (Titanium Dioxide), Magnesium Hydroxide, CI 15850, CI 77492 (Iron Oxides), Silica, Tin Oxide | 2,00 |
| Nailsyn^{®} Sterling 60 Silver | Merck KGaA/Rona^{®} | CI 77163 (Bismuth Oxychloride), Butyl Acetate, Nitrocellulose, Isopropyl Alcohol, Ethyl Acetate, Stearalkonium Hectorite | 1,00 |
| Thixotrope Nagellack -Base 155 | Durlin/Bergerac NC | BUTYL ACETATE, ETHYL ACETATE NITROCELLULOSE, ACETYL TRIBUTYL CITRATE, PHTHALIC ANHYDRIDE/TRIMELLIT IC ANHYDRIDE/GLYCOLS COPOLYMER, ISOPROPYLALCOHOL, STEARALKONIUM HECTORITE, ADIPIC ACID/FUMARIC ACID/PHTHALIC ACID/TRICYCLODECAN E DIMETHANOL COPOLYMER, CITRIC ACID | 97,00 |

### Herstellung:

Das Pigment wird zusammen mit der Lackbase eingewogen, gut mit einem Spatel von Hand vermischt und anschließend 10 min bei 1000 Upm gerührt.

### Volumenmascara

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, CI 77891 (Titanium Dioxide), Magnesium Hydroxide, CI 15850, CI 77492 (Iron Oxides), Silica, Tin Oxide | 10,00 |
| Satin Mica | Merck KGaA/Rona^{®} | MICA | 2,00 |

### Phase B:

| **Rohstoff** | **Bezugsquelle** | **INCI** | **[%]** |
|---|---|---|---|
| Dow Corning 556 | Dow Corning | PHENYL TRIMETHICONE | 2,00 |
| Tegosoft CT | Degussa-Goldschmidt AG | CAPRYLIC CAPRIC TRIGLYCERIDE | 2,50 |
| Syncrowachs HRC | Croda GmbH | TRIBEHENIN | 3,50 |
| Tegin M | Degussa-Goldschmidt AG | GLYCERYL STEARATE | 3,50 |
| Bienenwachs | Merck KGaA/Rona^{®} | CERA ALBA (BEESWAX) | 3,00 |
| Stearinsäure | Merck KGaA/Rona^{®} | STEARIC ACID | 5,00 |
| Phenonip | Nipa Laboratorien GmbH | PHENOXYETHANOL, BUTYLPARABEN, ETHYLPARABEN, PROPYLPARABEN, METHYLPARABEN | 0,80 |
| RonaCare^{®} Tocopherolacetat | Merck KGaA/Rona^{®} | TOCOPHEROL-ACETAT | 0,50 |
| Dermacryl79 | Amerchol | ACRYLATES/OCTYL-ACRYLAMIDE COPOLYMER | 3,50 |

### Phase C

| **Rohstoff** | **Bezugsquelle** | **INCI** | **[%]** |
|---|---|---|---|
| Wasser (demineralisiert) | | AQUA (WATER) | 59,15 |
| AMP Ultra PC 1000 | Angus Chemie GmbH | AMINOMETHYL PROPANOL | 1,25 |
| 1,3-Butandiol | Merck KGaA/Rona^{®} | BUTYLENE GLYCOL | 1,00 |
| RonaCare^{®} Biotin Plus | Merck KGaA/Rona^{®} | UREA, DISODIUM PHOSPHATE, BIOTIN, CITRIC ACID | 0,50 |

### Phase D

| **Rohstoff** | **Bezugsquelle** | **INCI** | **[%]** |
|---|---|---|---|
| Germall 115 | ISP Global Technologies | IMIDAZOLIDINYL UREA | 0,30 |
| Wasser (demineralisiert) | | AQUA (WATER) | 1,50 |

### Herstellung:

Alle Bestandteile der Phase B außer Demacryl 79 zusammen bei ca. 85 °C aufschmelzen, unter Rühren Demacryl 79 zugeben und 20 min rühren lassen bis alles homogen verteilt ist. Die Bestandteile der Phase C auf ca 85 °C erhitzen. Das rote Pigment der Phase A in Phase C einrühren. Phase C zu Phase B geben, weiter rühren und 1 min mit dem Ultra-Turrax T25 bei 8000 Upm homogenisieren. Unter rühren abkühlen lassen und bei 40 °C Phase D zufügen.

### Seife

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, Cl 77891 (Titanium Dioxide), Magnesium Hydroxide, Cl 15850, Cl 77492 (Iron Oxides), Silica, Tin Oxide | 1,50 |
| Ronastar^{®} Noble Sparks | Merck KGaA/Rona^{®} | Calcium Aluminum Borosilicate, Silica, Cl 77891 (Titanium Dioxide), Tin Oxide | 0,50 |
| Transparente Seifenbase | Jean Charles (USA) | SODIUM PALMATE, SODIUM LAURETH SULFATE, SODIUM STEARATE, SODIUM MYRISTATE, SODIUM COCOYL ISETHIONATE, TRIETHANOLAMINE, AQUA (WATER), GLYCERIN, SORBITOL, PROPYLENE GLYCOL, FRAGRANCE | 98,00 |

### Herstellung:

Alle Bestandteile werden homogen gemischt.

### Cheek & Eye Color

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, Cl 77891 (Titanium Dioxide), Magnesium Hydroxide, Cl 15850, CI 77492 (Iron Oxides), Silica, Tin Oxide | 13,00 |
| Satin Mica | Merck KGaA/Rona^{®} | MICA | 10,80 |
| Eusolex^{®} T-S | Merck KGaA/Rona^{®} | TITANIUM DIOXIDE, ALUMINA, STEARIC ACID | 5,00 |
| Talc | Merck KGaA/Rona^{®} | TALC | 4,00 |
| Timiron^{®} Arctic Silver | Merck KGaA/Rona^{®} | Cl 77891 (TITANIUM DIOXIDE), MICA, SILICA | 4,00 |
| Mica Black | Merck KGaA/Rona^{®} | Cl 77499 (IRON OXIDES), MICA, Cl 77891 (TITANIUM DIOXIDE) | 1,80 |
| Ronastar^{®} Silver | Merck KGaA/Rona^{®} | CALCIUM ALUMINUM BOROSILICATE,SILICA, Cl 77891 (TITANIUM DIOXIDE), TIN OXIDE | 0,40 |
| Unipure Blue LC 680 | Les Colorants Wackherr | CI 77007 (ULTRAMARINE BLUE) | 1,00 |

### Phase B:

| **Rohstoff** | **Bezugsquelle** | **INCI** | **[%]** |
|---|---|---|---|
| Crodamol PMP | Croda GmbH | PPG-2 MYRISTYL ETHER PROPIONATE | 24,40 |
| Miglyol 812 N | Sasol Germany GmbH | CAPRYLIC/CAPRIC TRIGLYCERIDE | 12,00 |
| Syncrowax HGLC | Croda GmbH | C18-36 ACID TRIGLYCERIDE | 8,00 |
| Myritol 331 | Cognis GmbH | COCOGLYCERIDES | 7,90 |
| Kester Wachs K 82 P | Koster Keunen Holland BV | SYNTHETIC WAX | 5,00 |
| Antaron V-216 | ISP Global Technologies | PVP/HEXADECENE COPOLYMER | 2,00 |

### Phase C

| **Rohstoff** | **Bezugsquelle** | **INCI** | **[%]** |
|---|---|---|---|
| Oxynex^{®} K liquid | Merck KGaA/Rona^{®} | PEG-8, TOCOPHEROL, ASCORBYL PALMITATE, ASCORBIC ACID, CITRIC ACID | 0,10 |
| Phenonip | Nipa Laboratorien GmbH | PHENOXYETHANOL, BUTYLPARABEN, ETHYLPARABEN, PROPYLPARABEN, METHYLPARABEN | 0,60 |

### Herstellung:

Phase B auf ca. 85°C erhitzen bis alles geschmolzen ist und auf 75°C abkühlen. Unter Rühren werden nun die Inhaltsstoffe der Phase A und C zugegeben und die fertige Masse bei 75°C abgefüllt.

### Shampoo

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, Cl 77891 (Titanium Dioxide), Magnesium Hydroxide, CI 15850, CI 77492 (Iron Oxides), Silica, Tin Oxide | 0,20 |
| Carbopol ETD 2020 | Noveon | ACRYLATES/C10-30 ALKYL ACRYLATE CROSSPOLYMER | 0,90 |
| Wasser, demineralisiert | | AQUA (WATER) | 63,40 |

### Phase B:

| **Rohstoff** | **Bezugsquelle** | **INCI** | **[%]** |
|---|---|---|---|
| Triethanolamin | | TRIETHANOLAMINE | 0,90 |
| Wasser, demineralisiert | | AQUA (WATER) | 10,00 |

### Phase C

| **Rohstoff** | **Bezugsquelle** | **INCI** | **[%]** |
|---|---|---|---|
| Plantacare 2000 UP | Cognis GmbH | DECYL GLUCOSIDE | 20,00 |
| Texapon ASV 50 | Cognis GmbH | SODIUM LAURETH SULFATE, SODIUM LAURETH-8 SULFATE, MAGNESIUM LAURETH SULFATE,MAGNESIUM LAURETH-8 SULFATE, SODIUM OLETH SULFATE, MAGNESIUM OLETH SULFATE | 4,35 |
| Bronidox L | Cognis GmbH | PROPYLENE GLYCOL, 5-BROMO-5-NITRO-1,3- DIOXANE | 0,20 |
| Parfümöl 200 524 | Fragrance Resources | PARFUM | 0,05 |

### Herstellung:

Für Phase A das Pigment in das Wasser einrühren. Mit einigen Tropfen Citronensäure (10%ig) ansäuern um die Viskosität zu vermindern und das Carbopol unter Rühren langsam einstreuen. Nach vollständiger Lösung langsam Phase B zugeben. Nacheinander werden nun die Bestandteile der Phase C zugegeben. Den pH-Wert auf 6,0 - 6,5 einstellen.

### Haargel

### Phase A

| **Rohstoff** | **Bezugsquelle** | **INCI** | **[%]** |
|---|---|---|---|
| rotes Pigment gemäß Beispiel 2 | Merck KGaA/Rona^{®} | Calcium Aluminum Borosilicate, CI 77891 (Titanium Dioxide), Magnesium Hydroxide, CI 15850, CI 77492 (Iron Oxides), Silica, Tin Oxide | 0,65 |
| Ronastar^{®} Red Sparks | Merck KGaA/Rona^{®} | CALCIUM ALUMINUM BOROSILICATE, CI 77891 (TITANIUM DIOXIDE), SILICA, TIN OXIDE | 2,00 |
| Carbopol Ultrez 21 | Noveon | ACRYLATES/C10-30 ALKYL ACRYLATE CROSSPOLYMER | 0,90 |
| Wasser, demineralisiert | | AQUA (WATER) | 50,35 |

### Phase B:

| **Rohstoff** | **Bezugsquelle** | **INCI** | **[%]** |
|---|---|---|---|
| Luviskol K 30 Pulver | BASF AG | PVP | 2,00 |
| Germaben II | ISP Global Technologies | PROPYLENE GLYCOL, DIAZOLIDINYL UREA, METHYLPARABEN, PROPYLPARABEN | 1,00 |
| Triethanolamin | | TRIETHANOLAMINE | 2,16 |
| Wasser, demineralisiert | | AQUA (WATER) | 4094 |

### Herstellung:

Die Perlglanzpigmente im Wasser der Phase A dispergieren und das Carbopol unter Rühren einstreuen. Nach vollständiger Lösung die vorgelöste Phase B langsam einrühren.

## Patentansprüche

1. Pigment umfassend ein plättchenförmiges Substrat, **dadurch gekennzeichnet, dass** das Substrat ausgewählt ist aus Glasplättchen, SiO₂-Plättchen Al₂O₃-Plättchen, künstlichem oder natürlichem plättchenförmigen Eisenoxid, künstlichem oder natürlichem Graphit und/oder plättchenförmigen Metallen und sich auf dem Substrat eine Anion-bindende Schicht umfassend ein schichtförmiges Doppelhydroxid (LDH) befindet, wobei die Anion-bindende Schicht ein Anion-bildendes organisches, anorganisches und/oder metallorganisches Farbmittel aus der Gruppe C.I. Acid-Farbstoffe, C.I. Reaktiv-Farbstoffe C.I. Direkt-Farbstoffe, C.I. Mordant-Farbstoffe, C.I. Solubilised Sulphur Red 11, Fluoreszenz-Farbstoffe, FD&C Yellow 5 (Tartrazin), FD&C Yellow 6 (Sunset Yellow FCF), FD&C Yellow 10, FD&C Red 3 (Erythrosin), FD&C Red 6 (Litholrubin B), FD&C Red 7 (Litholrubin BN), FD&C Red 21, FD&C Red 27, FD&C Red 28 (Floxine B), FD&C Red 33, C.I. Natural Red 33, FD&C Red 36, FD&C Red 40, Carmine, FD&C Blue 1 (Brilliant Blue FCF), C.I. Natural Green 3 (E141), FD&C Blue, FD&C Black 1 (Brilliant Black) enthält.

2. Pigment nach Anspruch 1, **dadurch gekennzeichnet, dass** das plättchenförmige Substrat Glasplättchen sind, die mit einer Schicht aus SiO₂ mit einer Schichtdicke von 5-350 nm beschichtet sind.

3. Pigment nach Anspruch 1 oder 2 , **dadurch gekennzeichnet, dass** das plättchenförmige Substrat mit Ionen oder Elementen dotiert ist.

4. Pigment gemäß einem oder mehreren der Ansprüche 1 bis 3, **dadurch gekennzeichnet, dass** die Anion-bindende Schicht ein schichtförmiges Doppelhydroxid der allgemeinen Formel
M²⁺₁₋ₓM³⁺ₓ(OH)₂(Zⁿ⁻)_{x/n} · mH₂O
umfasst, mit 0.2 < x < 0.33, wobei
M³⁺ ausgewählt ist aus Al³⁺, Cr³⁺, Fe³⁺, Ga³⁺, In³⁺, Y³⁺, La³⁺ und/oder Ce³⁺ und
M²⁺ ausgewählt ist aus Ba²⁺, Ca²⁺, Cu²⁺, Mg²⁺, Sr²⁺ und/oder Zn²⁺,
Z ein Gegenion der Metall-Salze und/oder ein Anion- oder ein Anionengemisch der Anion-bildenden organischen, anorganischen und/oder metallorganischen Farbmittel bedeutet, mit n für die Ladungszahl des Anions.

5. Pigment gemäß Anspruch 4, **dadurch gekennzeichnet, dass** M³⁺ = Al³⁺, Fe³⁺ ist und M²⁺ = Mg²⁺, Ca²⁺ oder Zn²⁺ ist.

6. Pigment gemäß einem oder mehreren der Ansprüche 1 bis 5, **dadurch gekennzeichnet, dass** das Doppelhydroxid ausgewählt ist aus Mg_{0.67}Al_{0.33}(OH)₂(Zⁿ⁻)_{0.33/n} mH₂O und Mg_{0.67}Fe_{0.33}(OH)₂(Zⁿ⁻)_{0.33/n} · mH₂O, Zn_{0.67}Al_{0.33}(OH)₂(Zⁿ⁻)_{0.33/n} · mH₂O und Zn_{0.67}Fe_{0.33}(OH)₂(Zⁿ⁻)_{0.33/n} · mH₂O, Ca_{0.67}Al_{0.33}(OH)₂(Zⁿ⁻)_{0.33/n} · mH₂O und Ca_{0.67}Fe_{0.33}(OH)₂(Zⁿ⁻)_{0.33/n} · mH₂O.

7. Pigment gemäß einem oder mehreren der Ansprüche 1 bis 6, **dadurch gekennzeichnet, dass** die Schichtdicke der Anion-bindenden Schicht 0.5-500 nm beträgt.

8. Pigment gemäß einem oder mehreren der Ansprüche 1 bis 7, **dadurch gekennzeichnet, dass** der Anteil der Anion-bildenden organischen und/oder anorganischen Farbmittel 0.01 bis 30 Gew.-%, insbesondere 0.5-10 Gew.-%, bezogen auf Gesamtpigment, beträgt.

9. Pigment gemäß einem oder mehreren der Ansprüche 1 bis 8, **dadurch gekennzeichnet, dass** das Farbmittel ausgewählt ist aus C.I. Acid Yellow 13, C.I. Acid Yellow 17, C.I. Acid Yellow 23, C.I. Acid Yellow 25, C.I. Acid Yellow 36, C.I. Acid Yellow 38, C.I. Acid Yellow 42, C.I. Acid Yellow 44, C.I. Acid Yellow 56, C.I. Acid Yellow 65, C.I. Acid Yellow 76, C.I. Acid Yellow 127, C.I. Acid Orange 7, C.I. Acid Orange 10, C.I. Acid Orange 19, C.I. Acid Orange 65, C.I. Acid Orange 67, C.I. Acid Red 1, C.I. Acid Red 13, C.I. Acid Red 14, C.I. Acid Red 32, C.I. Acid Red 37, C.I. Acid Red 38, C.I. Acid Red 42, C.I. Acid Red 88, C.I. Acid Red 119, C.I. Acid Red 131, C.I. Acid Red 138, C.I. Acid Red 154, C.I. Acid Red 249, C.I. Acid Red 299, C.I. Acid Violet 14, C.I. Acid Violet 42, C.I. Acid Violet 43, C.I. Acid Blue 25, C.I. Acid Blue 40, C.I. Acid Blue 43, C.I. Acid Blue 62, C.I. Acid Blue 92, C.I. Acid Blue 113, C.I. Acid Blue 117, C.I. Acid Blue 129, C.I. Acid Green 1, C.I. Acid Green 25, C.I. Acid Green 41, C.I. Acid Black 1, C.I. Acid Black 24, C.I. Acid Black 26, C.I. Acid Black 48, C.I. Acid Black 210, C.I. Acid Black 234, C.I. Acid Brown 14, C.I. Acid Brown 20, C.I. Reactive Yellow 4, C.I. Reactive Yellow 17, C.I. Reactive Orange 1, C.I. Reactive Red 8, C.I. Reactive Red 12, C.I. Reactive Red 23, C.I. Reactive Blue 15, C.I. Reactive Blue 19, C.I. Reactive Blue 216, C.I. Reactive Black 5, C.I. Reactive Black 8, C.I. Reactive Black 31, C.I. Direct Yellow 12, C.I. Direct Yellow 27, C.I. Direct Yellow 29, C.I. Direct Yellow 50, C.I. Direct Yellow 86, C.I. Direct Orange 26, C.I. Direct Red 23, C.I. Direct Red 75, C.I. Direct Red 76, C.I. Direct Red 79, C.I. Direct Red 80, C.I. Direct Red 81, C.I. Direct Red 250, C.I. Direct Blue 78, C.I. Direct Blue 86, C.I. Direct Blue 93, C.I. Direct Blue 106, C.I. Direct Green 26, C.I. Direct Black 19, C.I. Direct Black 22, C.I. Direct Black 51, C.I. Direct Black 150, C.I. Direct Black 151, C.I. Direct Black 166, C.I. Direct Black 168, C.I. Mordant Yellow 1, C.I. Mordant Yellow 5, C.I. Mordant Yellow 30, C.I. Mordant Red 7, C.I. Mordant Red 19, C.I. Mordant Red 30, C.I. Mordant Blue 7, C.I. Mordant Blue 13, C.I. Mordant Black 3, C.I. Mordant Black 9, C.I. Mordant Black 11, C.I. Mordant Brown 33, C.I. Mordant Brown 48, aber auch C.I. Solubilised Sulphur Red 11, C.I. Basic Yellow 40, C.I. Basic Red 12 oder C.I. Solvent Yellow 94.

10. Pigment gemäß einem oder mehreren der Ansprüche 1 bis 9, **dadurch gekennzeichnet**, däss auf dem Pigment zusätzlich eine stabilisierende anorganische und/oder organische Beschichtung aufgebracht ist.

11. Verfahren zur Herstellung eines Pigmentes gemäß einem oder mehreren der Ansprüche 1 bis 10 , wobei eine Suspension von plättchenförmigem Substrat, Metallkation-Salzen, Farbmittelsalzen sowie Laugen und/oder Laugenvorstufen in einem Lösungsmittel oder Lösungsmittelgemisch bei einer Temperatur von 10-120°C gerührt wird, so dass sich auf dem Substrat die Anion-bindende Schicht bildet, wobei die Anion-bindende Schicht Anion-bildende organische, anorganische und/oder metallorganische Farbmittel enthält, anschließend das Produkt abgetrennt, gewaschen, getrocknet und gegebenenfalls gesiebt wird.

12. Verfahren nach Anspruch 11, **dadurch gekennzeichnet, dass** eine Lösung der Metallkation-Salze zu einer Suspension aus plättchenförmigem Substrat, Farbmittelsalzen sowie Laugen und/oder Laugenvorstufen zugegeben wird.

13. Verfahren zur Herstellung eines Pigmentes gemäß einem oder mehreren der Ansprüche 1 bis 10, wobei eine Suspension von plättchenförmigem Substrat, Metallkation-Salzen sowie Laugen und/oder Laugenvorstufen in einem Lösungsmittel oder Lösungsmittelgemisch bei einer Temperatur von 10-120°C gerührt wird, so dass sich auf dem Substrat die Anion-bindende Schicht bildet, danach das Zwischenprodukt abgetrennt, gewaschen, getrocknet und gegebenenfalls gesiebt wird und dieses Zwischenprodukt anschließend zu einer Lösung der Anion-bildenden organischen, anorganischen und/oder metallorganischen Farbmittel unter Rühren hinzu gegeben wird.

14. Verfahren gemäß Anspruch 13, **dadurch gekennzeichnet, dass** das Produkt mit der auf dem Substrat gebildeten Anion-bindenden Schicht vor Zugabe zu der Lösung der Anion-bildenden organischen und/oder anorganischen Farbmittel bei Temperaturen von 300-600°C kalciniert wird.

15. Verwendung eines Pigmentes gemäß einem oder mehreren der Ansprüche 1 bis 10 in Kosmetika, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

16. Verwendung gemäß Anspruch 15, **dadurch gekennzeichnet**, das das Pigment gemäß einem oder mehreren der Ansprüche 1 bis 10 als Mischung mit organischen und/oder anorganischen Farbstoffen und/oder Pigmenten vorliegt.

17. Kosmetika, Lacke, Farben, Kunststoffe, Folien, Dokumente und Ausweise, Saatgut, Lebensmittel oder Arzneimittelüberzüge sowie Pigmentpräparationen und Trockenpräparate enthaltend ein Pigment gemäß einem oder mehreren der Ansprüche 1 bis 10.

## Claims

1. Pigment comprising a flake-form substrate, **characterised in that** the substrate is selected from glass flakes, SiO₂ flakes, Al₂O₃ flakes, synthetic or natural flake-form iron oxide, synthetic or natural graphite and/or flake-form metals and an anion-binding layer comprising a layered double hydroxide (LDH) is located on the substrate, where the anion-binding layer comprises an anion-forming organic, inorganic and/or organometallic colorant from the group C.I. acid dyes, C.I. reactive dyes, C.I. direct dyes, C.I. mordant dyes, C.I. Solubilised Sulphur Red 11, fluorescent dyes, FD&C Yellow 5 (tartrazine), FD&C Yellow 6 (Sunset Yellow FCF), FD&C Yellow 10, FD&C Red 3 (erythrosine), FD&C Red 6 (Litholrubin B), FD&C Red 7 (Litholrubin BN), FD&C Red 21, FD&C Red 27, FD&C Red 28 (Floxine B), FD&C Red 33, C.I. Natural Red 33, FD&C Red 36, FD&C Red 40, carmine, FD&C Blue 1 (Brilliant Blue FCF), C.I. Natural Green 3 (E141), FD&C Blue, FD&C Black 1 (Brilliant Black).

2. Pigment according to Claim 1, **characterised in that** the flake-form substrate comprises glass flakes which are coated with a layer of SiO₂ having a layer thickness of 5-350 nm.

3. Pigment according to Claim 1 or 2, **characterised in that** the flake-form substrate is doped with ions or elements.

4. Pigment according to one or more of Claims 1 to 3, **characterised in that** the anion-binding layer comprises a layered double hydroxide of the general formula
M²⁺₁₋ₓM³⁺ₓ(OH)₂(Zⁿ⁻)_{x/n} · mH₂O
where 0.2 < x < 0.33, where
M³⁺ is selected from Al³⁺, Cr³⁺, Fe³⁺, Ga³⁺, In³⁺, Y³⁺, La³⁺ and/or Ce³⁺ and
M²⁺ is selected from Ba²⁺, Ca²⁺, Cu²⁺, Mg²⁺, Sr²⁺ and/or Zn²⁺,
Z denotes a counterion of the metal salts and/or an anion or anion mixture of the anion-forming organic, inorganic and/or organometallic colorants, where n stands for the charge number of the anion.

5. Pigment according to Claim 4, **characterised in that** M³⁺ = Al³⁺ or Fe³⁺ and M²⁺ = Mg²⁺, Ca²⁺ or Zn²⁺.

6. Pigment according to one or more of Claims 1 to 5, **characterised in that** the double hydroxide is selected from Mg_{0.67}Al_{0.33}(OH)₂(Zⁿ⁻)_{0.33/n} · mH₂O and Mg_{0.67}Fe_{0.33}(OH)₂(Zⁿ⁻)_{0.33/n} · mH₂O, Zn_{0.67}Al_{0.33}(OH)₂(Zⁿ⁻)_{0.33/n} · mH₂O and Zn_{0.67}Fe_{0.33}(OH)₂(Zⁿ⁻)_{0.33/n} · mH₂O, Ca_{0.67}Al_{0.33}(OH)₂(Zⁿ⁻)_{0.33/n} · mH₂O and Ca_{0.67}Fe_{0.33}(OH)₂(Zⁿ⁻)_{0.33/n} · mH₂O.

7. Pigment according to one or more of Claims 1 to 6, **characterised in that** the layer thickness of the anion-binding layer is 0.5-500 nm.

8. Pigment according to one or more of Claims 1 to 7, **characterised in that** the proportion of the anion-forming organic and/or inorganic colorants is 0.01 to 30% by weight, in particular 0.5-10% by weight, based on the entire pigment.

9. Pigment according to one or more of Claims 1 to 8, **characterised in that** the colorant is selected from C.I. Acid Yellow 13, C.I. Acid Yellow 17, C.I. Acid Yellow 23, C.I. Acid Yellow 25, C.I. Acid Yellow 36, C.I. Acid Yellow 38, C.I. Acid Yellow 42, C.I. Acid Yellow 44, C.I. Acid Yellow 56, C.I. Acid Yellow 65, C.I. Acid Yellow 76, C.I. Acid Yellow 127, C.I. Acid Orange 7, C.I. Acid Orange 10, C.I. Acid Orange 19, C.I. Acid Orange 65, C.I. Acid Orange 67, C.I. Acid Red 1, C.I. Acid Red 13, C.I. Acid Red 14, C.I. Acid Red 32, C.I. Acid Red 37, C.I. Acid Red 38, C.I. Acid Red 42, C.I. Acid Red 88, C.I. Acid Red 119, C.I. Acid Red 131, C.I. Acid Red 138, C.I. Acid Red 154, C.I. Acid Red 249, C.I. Acid Red 299, C.I. Acid Violet 14, C.I. Acid Violet 42, C.I. Acid Violet 43, C.I. Acid Blue 25, C.I. Acid Blue 40, C.I. Acid Blue 43, C.I. Acid Blue 62, C.I. Acid Blue 92, C.I. Acid Blue 113, C.I. Acid Blue 117, C.I. Acid Blue 129, C.I. Acid Green 1, C.I. Acid Green 25, C.I. Acid Green 41, C.I. Acid Black 1, C.I. Acid Black 24, C.I. Acid Black 26, C.I. Acid Black 48, C.I. Acid Black 210, C.I. Acid Black 234, C.I. Acid Brown 14, C.I. Acid Brown 20, C.I. Reactive Yellow 4, C.I. Reactive Yellow 17, C.I. Reactive Orange 1, C.I. Reactive Red 8, C.I. Reactive Red 12, C.I. Reactive Red 23, C.I. Reactive Blue 15, C.I. Reactive Blue 19, C.I. Reactive Blue 216, C.I. Reactive Black 5, C.I. Reactive Black 8, C.I. Reactive Black 31, C.I. Direct Yellow 12, C.I. Direct Yellow 27, C.I. Direct Yellow 29, C.I. Direct Yellow 50, C.I. Direct Yellow 86, C.I. Direct Orange 26, C.I. Direct Red 23, C.I. Direct Red 75, C.I. Direct Red 76, C.I. Direct Red 79, C.I. Direct Red 80, C.I. Direct Red 81, C.I. Direct Red 250, C.I. Direct Blue 78, C.I. Direct Blue 86, C.I. Direct Blue 93, C.I. Direct Blue 106, C.I. Direct Green 26, C.I. Direct Black 19, C.I. Direct Black 22, C.I. Direct Black 51, C.I. Direct Black 150, C.I. Direct Black 151, C.I. Direct Black 166, C.I. Direct Black 168, C.I. Mordant Yellow 1, C.I. Mordant Yellow 5, C.I. Mordant Yellow 30, C.I. Mordant Red 7, C.I. Mordant Red 19, C.I. Mordant Red 30, C.I. Mordant Blue 7, C.I. Mordant Blue 13, C.I. Mordant Black 3, C.I. Mordant Black 9, C.I. Mordant Black 11, C.I. Mordant Brown 33, C.I. Mordant Brown 48, but also C.I. Solubilised Sulphur Red 11, C.I. Basic Yellow 40, C.I. Basic Red 12 or C.I. Solvent Yellow 94.

10. Pigment according to one or more of Claims 1 to 9, **characterised in that** a stabilising inorganic and/or organic coating is additionally applied to the pigment.

11. Process for the preparation of a pigment according to one or more of Claims 1 to 10, where a suspension of flake-form substrate, metal cation salts, colorant salts and lyes and/or lye precursors is stirred at a temperature of 10-120°C in a solvent or solvent mixture so that the anion-binding layer forms on the substrate, where the anion-binding layer comprises anion-forming organic, inorganic and/or organometallic colorants, the product is subsequently separated off, washed, dried and optionally sieved.

12. Process according to Claim 11, **characterised in that** a solution of the metal cation salts is added to a suspension of flake-form substrate, colorant salts and lyes and/or lye precursors.

13. Process for the preparation of a pigment according to one or more of Claims 1 to 10, where a suspension of flake-form substrate, metal cation salts and lyes and/or lye precursors is stirred at a temperature of 10-120°C in a solvent or solvent mixture so that the anion-binding layer forms on the substrate, the intermediate is then separated off, washed, dried and optionally sieved, and this intermediate is subsequently added to a solution of the anion-forming organic, inorganic and/or organometallic colorants with stirring.

14. Process according to Claim 13, **characterised in that** the product is calcined at temperatures of 300-600°C with the anion-binding layer formed on the substrate before addition to the solution of the anion-forming organic and/or inorganic colorants.

15. Use of a pigment according to one or more of Claims 1 to 10 in cosmetics, paints, coatings, plastics, films, in security printing, in security features in documents and identity cards, for colouring seed, for colouring foods or in medicament coatings and for the preparation of pigment compositions and dry preparations.

16. Use according to Claim 15, **characterised in that** the pigment according to one or more of Claims 1 to 10 is in the form of a mixture with organic and/or inorganic dyes and/or pigments.

17. Cosmetics, paints, coatings, plastics, films, documents and identity cards, seed, foods or medicament coatings and pigment compositions and dry preparations comprising a pigment according to one or more of Claims 1 to 10.

## Revendications

1. Pigment comprenant un substrat sous forme de paillettes, **caractérisé en ce que** le substrat est choisi parmi les paillettes de verre, les paillettes de SiO₂, les paillettes d'Al₂O₃, un oxyde de fer sous forme de paillettes synthétique ou naturel, le graphite synthétique ou naturel et/ou des métaux sous forme de paillettes et une couche fixatrice d'anions comprenant un hydroxyde double lamellaire (LDH) est située sur le substrat, où la couche fixatrice d'anions comprend un colorant formateur d'anions organique, inorganique et/ou organométallique issu du groupe des colorants acides C.I., des colorants réactifs C.I., des colorants directs C.I., des colorants mordançables C.I., C.I. Solubilised Sulphur Red 11, les colorants fluorescents, le FD&C Yellow 5 (tartrazine), le FD&C Yellow 6 (Sunset Yellow FCF), le FD&C Yellow 10, le FD&C Red 3 (érythrosine), le FD&C Red 6 (Litholrubine B), le FD&C Red 7 (Litholrubine BN), le FD&C Red 21, le FD&C Red 27, le FD&C Red 28 (Floxine B), le FD&C Red 33, le C.I. Natural Red 33, le FD&C Red 36, le FD&C Red 40, la carmine, le FD&C Blue 1 (Brilliant Blue FCF), le C.I. Natural Green 3 (E141), le FD&C Blue, le FD&C Black 1 (Brilliant Black).

2. Pigment selon la revendication 1, **caractérisé en ce que** le substrat sous forme de paillettes comprend des paillettes de verre qui sont revêtues d'une couche de SiO₂ ayant une épaisseur de couche de 5-350 nm.

3. Pigment selon la revendication 1 ou 2, **caractérisé en ce que** le substrat sous forme de paillettes est dopé par des ions ou des éléments.

4. Pigment selon l'une ou plusieurs parmi les revendications 1 à 3, **caractérisé en ce que** la couche fixatrice d'anions comprend un hydroxyde double lamellaire de formule générale
M²⁺₁₋ₓM³⁺ₓ(OH)₂(Zⁿ⁻)_{x/n} · mH₂O
où 0,2 < x < 0,33, où
M³⁺ est choisi parmi Al³⁺, Cr³⁺, Fe³⁺, Ga³⁺, In³⁺, Y³⁺, La³⁺ et/ou Ce³⁺ et
M²⁺ est choisi parmi Ba²⁺, Ca²⁺, Cu²⁺, Mg²⁺, Sr²⁺ et/ou Zn²⁺,
Z désigne un contre-ion des sels métalliques et/ou un anion ou mélange d'anions des colorants formateurs d'anions organiques, inorganiques et/ou organométalliques, où n représente le nombre de charge de l'anion.

5. Pigment selon la revendication 4, **caractérisé en ce que** M³⁺ = Al³⁺ ou Fe³⁺ et M²⁺ = Mg²⁺, Ca²⁺ ou Zn²⁺.

6. Pigment selon l'une ou plusieurs parmi les revendications 1 à 5, **caractérisé en ce que** l'hydroxyde double est choisi parmi Mg_{0,67}Al_{0.33}(OH)₂(Zⁿ⁻)_{0,33/n}, mH₂O et Mg_{0,67}Fe_{0,33}(OH)₂(Zⁿ⁻)_{0,33/n} · mH₂O, Zn_{0,67}Al_{0,33}(OH)₂(Zⁿ⁻)_{0,33/n} mH₂O et Zn_{0,67}Fe_{0,33}(OH)₂(Zⁿ⁻)_{0,33/n} · mH₂O, Ca_{0,67}Al_{0,33}(OH)₂(Zⁿ⁻)_{0,33/n} · mH₂O et Ca_{0,67}Fe_{0.33}(OH)₂(Zⁿ⁻)_{0,33/n} · mH₂O.

7. Pigment selon l'une ou plusieurs parmi les revendications 1 à 6, **caractérisé en ce que** l'épaisseur de couche de la couche fixatrice d'anions est de 0,5-500 nm.

8. Pigment selon l'une ou plusieurs parmi les revendications 1 à 7, **caractérisé en ce que** la proportion des colorants formateurs d'anions organiques et/ou inorganiques va de 0,01 à 30% en poids, en particulier 0,5-10% en poids, sur la base de la totalité du pigment.

9. Pigment selon l'une ou plusieurs parmi les revendications 1 à 8, **caractérisé en ce que** le colorant est choisi parmi C.I. Acid Yellow 13, C.I. Acid Yellow 17, C.I. Acid Yellow 23, C.I. Acid Yellow 25, C.I. Acid Yellow 36, C.I. Acid Yellow 38, C.I. Acid Yellow 42, C.I. Acid Yellow 44, C.I. Acid Yellow 56, C.I. Acid Yellow 65, C.I. Acid Yellow 76, C.I. Acid Yellow 127, C.I. Acid Orange 7, C.I. Acid Orange 10, C.I. Acid Orange 19, C.I. Acid Orange 65, C.I. Acid Orange 67, C.I. Acid Red 1, C.I. Acid Red 13, C.I. Acid Red 14, C.I. Acid Red 32, C.I. Acid Red 37, C.I. Acid Red 38, C.I. Acid Red 42, C.I. Acid Red 88, C.I. Acid Red 119, C.I. Acid Red 131, C.I. Acid Red 138, C.I. Acid Red 154, C.I. Acid Red 249, C.I. Acid Red 299, C.I. Acid Violet 14, C.I. Acid Violet 42, C.I. Acid Violet 43, C.I. Acid Blue 25, C.I. Acid Blue 40, C.I. Acid Blue 43, C.I. Acid Blue 62, C.I. Acid Blue 92, C.I. Acid Blue 113, C.I. Acid Blue 117, C.I. Acid Blue 129, C.I. Acid Green 1, C.I. Acid Green 25, C.I. Acid Green 41, C.I. Acid Black 1, C.I. Acid Black 24, C.I. Acid Black 26, C.I. Acid Black 48, C.I. Acid Black 210, C.I. Acid Black 234, C.I. Acid Brown 14, C.I. Acid Brown 20, C.I. Reactive Yellow 4, C.I. Reactive Yellow 17, C.I. Reactive Orange 1, C.I. Reactive Red 8, C.I. Reactive Red 12, C.I. Reactive Red 23, C.I. Reactive Blue 15, C.I. Reactive Blue 19, C.I. Reactive Blue 216, C.I. Reactive Black 5, C.I. Reactive Black 8, C.I. Reactive Black 31, C.I. Direct Yellow 12, C.I. Direct Yellow 27, C.I. Direct Yellow 29, C.I. Direct Yellow 50, C.I. Direct Yellow 86, C.I. Direct Orange 26, C.I. Direct Red 23, C.I. Direct Red 75, C.I. Direct Red 76, C.I. Direct Red 79, C.I. Direct Red 80, C.I. Direct Red 81, C.I. Direct Red 250, C.I. Direct Blue 78, C.I. Direct Blue 86, C.I. Direct Blue 93, C.I. Direct Blue 106, C.I. Direct Green 26, C.I. Direct Black 19, C.I. Direct Black 22, C.I. Direct Black 51, C.I. Direct Black 150, C.I. Direct Black 151, C.I. Direct Black 166, C.I. Direct Black 168, C.I. Mordant Yellow 1, C.I. Mordant Yellow 5, C.I. Mordant Yellow 30, C.I. Mordant Red 7, C.I. Mordant Red 19, C.I. Mordant Red 30, C.I. Mordant Blue 7, C.I. Mordant Blue 13, C.I. Mordant Black 3, C.I. Mordant Black 9, C.I. Mordant Black 11, C.I. Mordant Brown 33, C.I. Mordant Brown 48, mais aussi C.I. Solubilised Sulphur Red 11, C.I. Basic Yellow 40, C.I. Basic Red 12 ou C.I. Solvent Yellow 94.

10. Pigment selon l'une ou plusieurs parmi les revendications 1 à 9, **caractérisé en ce que** qu'un revêtement stabilisant inorganique et/ou organique est en outre appliqué au pigment.

11. Procédé de préparation d'un pigment selon l'une ou plusieurs parmi les revendications 1 à 10, où une suspension de substrat sous forme de paillettes, de sels de cations métalliques, de sels de colorants et de lessives et/ou de précurseurs de lessive est agitée à une température de 10-120°C dans un solvant ou un mélange de solvants de sorte que la couche fixatrice d'anions se forme sur le substrat, où la couche fixatrice d'anions comprend des colorants formateurs d'anions organiques, inorganiques et/ou organométalliques, le produit est ensuite séparé, lavé, séché et éventuellement tamisé.

12. Procédé selon la revendication 11, **caractérisé en ce qu'**une solution des sels de cations métalliques suspension est ajoutée à une suspension de substrat sous forme de paillettes, de sels de colorants et de lessives et/ou de précurseurs de lessive.

13. Procédé de préparation d'un pigment selon l'une ou plusieurs parmi les revendications 1 à 10, où une suspension de substrat sous forme de paillettes, de sels de cations métalliques et de lessives et/ou de précurseurs de lessive est agitée à une température de 10-120°C dans un solvant ou un mélange de solvants de sorte que la couche fixatrice d'anions se forme sur le substrat, l'intermédiaire est ensuite séparé, lavé, séché et éventuellement tamisé, et cet intermédiaire est ensuite ajouté à une solution des colorants formateurs d'anions organiques, inorganiques et/ou organométalliques sous agitation.

14. Procédé selon la revendication 13, **caractérisé en ce que** le produit est calciné à des températures de 300-600°C, la couche fixatrice d'anions étant formée sur le substrat avant addition à la solution des colorants formateurs d'anions organiques et/ou inorganiques.

15. Utilisation d'un pigment selon l'une ou plusieurs parmi les revendications 1 à 10, dans les produits cosmétiques, les peintures, les revêtements, les matières plastiques, les films, dans l'impression de sécurité, dans les dispositifs de sécurité dans des documents et des cartes d'identité, pour la coloration de semences, pour la coloration d'aliments ou dans des enrobages de médicaments et pour la préparation de compositions de pigments et de préparations sèches.

16. Utilisation selon la revendication 15, **caractérisée en ce que** le pigment selon l'une ou plusieurs parmi les revendications 1 à 10 se trouve sous la forme d'un mélange avec des colorants et/ou des pigments organiques et/ou inorganiques.

17. Produits cosmétiques, peintures, revêtements, matières plastiques, films, documents et cartes d'identité, semences, aliments ou enrobages de médicaments, et compositions de pigments et préparations sèches comprenant un pigment selon l'une ou plusieurs parmi les revendications 1 à 10.
